(19) Europäisches Patentamt
European Patent Office
Office européen des brevets

(11) **EP 4 729 170 A1**

(12) **EUROPEAN PATENT APPLICATION**

(43) Date of publication:
**22.04.2026  Bulletin 2026/17**

(21) Application number: **24207402.9**

(22) Date of filing: **18.10.2024**

(51) International Patent Classification (IPC):
**B01J 23/80** (2006.01)      **B01J 37/02** (2006.01)
**B01J 37/03** (2006.01)      **C07C 29/156** (2006.01)
**C07C 31/08** (2006.01)

(52) Cooperative Patent Classification (CPC):
(C-Sets available)
**B01J 37/0236; B01J 23/002; B01J 23/80;
B01J 29/46; B01J 35/45; B01J 35/613;
B01J 35/633; B01J 35/643; B01J 35/647;
B01J 37/0201; B01J 37/031; B01J 37/18;
C07C 29/156;** B01J 2235/30; B01J 2523/00

(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Kemijski Institut
1000 Ljubljana (SI)**

(72) Inventors:
• **KOSTYNIUK, Andrii
1000 Ljubljana (SL)**
• **LIKOZAR, Blaz
1000 Ljubljana (SL)**

(74) Representative: **Weickmann & Weickmann
PartmbB
Postfach 860 820
81635 München (DE)**

(54) **CO2 HYDROGENATION TO HIGH-CONCENTRATED ETHANOL OVER TRIMETALLIC ALKALI-TREATED CATALYSTS**

(57)    The present invention relates to the direct hydrogenation of $CO_2$ into ethanol and higher alcohols using optimized xCs/CuFeZn or xRb/CuFeZn catalysts. This document showcases not only their enhanced performance and high selectivity but also offers valuable insights into catalyst design and the underlying reaction mechanisms. In addition, various factors such as Cs loading, reaction temperature, and time-on-stream were investigated.

**Fig. 7**

**(a)**

EP 4 729 170 A1

(52) Cooperative Patent Classification (CPC): (Cont.)

C-Sets
**C07C 29/156, C07C 31/08;**
B01J 2523/00, B01J 2523/14, B01J 2523/17,
B01J 2523/27, B01J 2523/842

**Description**

**Introduction**

Every day, large amounts of greenhouse gases (GHGs) are emitted into the atmosphere, contributing to global warming, with $CO_2$ making up 72% of total GHG emissions [1]. This dominance is mainly due to the extensive burning of fossil fuels, and $CO_2$ emissions have been steadily increasing, reaching 33.9 billion metric tons globally in 2018 [2]. To combat this, carbon capture and utilization (CCU) and carbon capture and storage (CCS) are crucial strategies, with CCU being seen as a particularly promising solution for reducing $CO_2$ emissions and addressing climate change [3]. Utilizing $CO_2$ as a resource for chemical processes, alongside renewable hydrogen production from water electrolysis, provides an environmentally sustainable approach to reducing $CO_2$ emissions while generating valuable products like CO, $CH_4$, light olefins, aromatics, dimethyl ether, formates, and alcohols. Among the various methods, one of the most promising is the direct thermocatalytic hydrogenation of $CO_2$ into ethanol using multifunctional heterogeneous catalysts. Ethanol can be synthesized through methods like ethylene hydration using a solid acid catalyst or fermentation of sugar, grain crops, and waste biomass. While fermentation is more sustainable and environmentally friendly than ethylene hydration, it faces limitations due to ethanol's toxicity to yeast, requiring distillation for higher concentrations, and raises ethical concerns about food versus fuel when using crops like corn and sugar cane [4].

The pursuit of methods for converting $CO_2$ into ethanol through hydrogenation is a significant area of interest, albeit one fraught with challenges in its development. Traditionally, noble-metal-based catalysts (such as Pd, Pt, and Rh) have been employed to overcome the initial hurdles involving C-O activation and subsequent C-C coupling in the hydrogenation of $CO_2$ to ethanol. However, the steep cost associated with these catalysts has prompted a shift from conventional approaches to the exploration of more abundant transition metals [5]. Cu-based catalysts demonstrate high activity in the synthesis of methanol from $CO_2$ or CO, yet they display reduced selectivity towards $C_{2+}$ oxygenates; concurrently, alkali metals play crucial roles as promoters in Cu-catalyzed ethanol synthesis from syngas, with selectivity ranking as Cs > K > Na > Li [6].

Recently, in a breakthrough study, Xu et al. [7] introduced a novel Cs-promoted Cu-Fe-Zn catalyst tailored for the precise synthesis of $C_{2+}$ alcohols. This catalyst demonstrated $C_{2+}$ alcohols STY of 1.47 mmol $g_{cat}^{-1}$ $h^{-1}$. This remarkable achievement is rooted in the intricate synergy between the Cs promoter, Cu-ZnO, and copper-iron carbide, forming interfaces within the material structure. The pivotal role of Cs is evident as it orchestrates the CO insertion reaction by fine-tuning the hydrogenation capabilities of the CuFeZn catalysts. However, despite this significant advancement, it is worth noting that the achieved STY is not yet suitable for large-scale applications. Yang and co-authors [8] subsequently prepared a CuFeZn catalyst using 2,6-pyridine dicarboxylic acid (PDA), achieving remarkable results, including the highest $C_{2+}OH/ROH$ fraction of 95.3% and a $C_{2+}$ alcohols STY of 58.2 mg/mL$_{cat/h}$, all while maintaining a $CO_2$ conversion rate of 10.6%. The authors attributed this exceptional catalytic performance to the coordination between PDA and the active metal ions, which facilitates electron transfer and the reduction of metallic oxides. Consequently, this coordination results in the even distribution of active components. Regrettably, it should be noted that despite its impressive performance, this catalyst exhibits a lower STY compared to the previous one utilizing Cs. Achieving efficient $CO_2$ conversion to ethanol remains challenging due to low selectivity, productivity, and insufficient catalyst stability. The direct conversion of $CO_2$ to ethanol, more complex than multi-step reactions, continues to deliver poor yields and selectivity, driving the search for innovative strategies in CO2 hydrogenation for ethanol production [5].

**Summary of the invention**

The present invention relates to the direct hydrogenation of $CO_2$ into ethanol and higher alcohols using optimized xCs/CuFeZn or xRb/CuFeZn catalysts. This document showcases not only their enhanced performance and high selectivity but also offers valuable insights into catalyst design and the underlying reaction mechanisms. In addition, various factors such as Cs loading, reaction temperature, and time-on-stream were investigated. The described catalysts have ranked among the top in terms of STY of ethanol in the literature. The xCs/CuFeZn and xRb/CuFeZn catalysts were extensively characterized using several advanced techniques, including BET (Brunauer-Emmett-Teller) surface area analysis, XRD (X-ray diffraction), XPS (X-ray photoelectron spectroscopy), SEM (Scanning electron microscopy), STEM (Scanning transmission electron microscopy), EDX (Energy-dispersive X-ray spectroscopy), and ICP-OES (Inductively coupled plasma optical emission spectroscopy). BET analysis revealed the surface area and pore distribution crucial for active site exposure. XRD identified the crystalline phases present in the catalyst, while XPS provided insights into the chemical states of the active elements, which are essential for catalytic performance. SEM, STEM, and EDX mapping offered detailed morphological insights and elemental distribution, highlighting the homogeneity and synergies among the catalyst components. Additionally, ICP-OES was employed to quantify the precise elemental composition of the catalyst, ensuring accurate assessment of Cs or Rb loading and its impact on catalytic behavior. These analyses collectively elucidated the underlying reaction mechanisms, guiding the optimization of the catalyst for enhanced $CO_2$ hydrogenation

to ethanol.

**Detailed description**

The present invention provides an improved hydrogenation catalyst comprising or essentially consisting of Cu-Fe-Zn mixed oxide particles loaded with Cs or Rb in an amount of 2-8 wt.%, based on the total weight of the loaded Cu-Fe-Zn mixed oxide particles. These catalyst particles demonstrate effective and selective synthesis of $C_{2+}$ alcohols through $CO_2$ hydrogenation with corresponding conversion.

Preferably, Cs or Rb is loaded in an amount of 3-6 mol %, more preferably 3.5-5 mol, based on the total weight of the loaded Cu-Fe-Zn mixed oxide particles. Especially preferred is a loading with about 4 mol % of Cs or Rb. 4% doping level induces an optimal electronic environment that may enhance the catalytic properties of the CuFeZn catalyst. Cs- or Rb-loading can be determined using SEM-EDX analysis.

Cu-Fe-Zn mixed oxide particles are essentially composed of Cu, Zn, and Fe oxides. Their respective amounts may differ. In preferred aspects, the molar ratio of Cu : Zn : Fe in the mixed oxide particles is in a range of 1 : 2-3 : 1-2, preferably about 1 : 2 : 1.

Based on the total weight of the Rb- or Cs-loaded Cu-Fe-Zn mixed oxide particles, Cu may be comprised in an amount of 10-25 wt.%, preferably 18-22 wt.%, more preferably about 20 wt.%. Zn may be comprised in an amount of 10-25 wt.%, preferably 12-16 wt.%, more preferably about 15 wt.%, and Fe may be comprised in an amount of 33-45 wt.%, preferably 35-38 wt.%, more preferably about 37 wt.%.

The catalyst of the invention is preferably a powder comprising or essentially consisting of particles with an average particle size in a range of 10-50 nm, more preferably 15-30 nm.

The Cu-Fe-Zn mixed oxide particles loaded with Cs or Rb are porous particles. In preferred aspects, they comprise mesopores and micropores. Mesopores are pores with diameters between 2 and 50 nm. Micropores are pores spaller than 2 nm in diameter. In preferred embodiments, the average pore diameter is in a range of 5-20 nm, more preferably 6-18 nm. This porosity yields an exceptional catalytic activity of the Cs- or Rb loaded particles. The incorporation of Cs or Rb leads to the formation of a more open and porous structure. The increased porosity is beneficial as it can enhance the accessibility of reactants to the active sites of the catalyst, potentially improving catalytic performance. Additionally, the surface of the particles appears smoother compared to the CuFeZn catalyst, which might influence the interaction with reactants.

According to another embodiment, the invention provides a method for preparing the hydrogenation catalyst described herein. Generally, the catalyst can be prepared in a two-step process or in a one-step process. "Two-step" means that Cu-Fe-Zn mixed oxide particles are prepared and subsequently loaded with Cs or Rb, whereas a "one-step" process directly yields Cs- or Rb-loaded particles.

Thus, a first method for preparing the hydrogenation catalyst comprises the steps of:

(i) providing Cu-Fe-Zn mixed oxide particles;
(ii) impregnating the Cu-Fe-Zn mixed oxide particles with a solution of a Cs salt or Rb salt, preferably an aqueous solution of CsNOa or RbNOa;
(iii) drying the impregnated particles, preferably by heating to a temperature in a range of 50-150°C, more preferably 60-130°C, in particular about 110°C;
(iv) calcination of the dried particles, preferably at a temperature in a range of 300-600°C, more preferably 350-500°C, in particular about 400°C.

Providing Cu-Fe-Zn mixed oxide particles in step (i) may comprise the following steps:

(i.1) coprecipitating Cu, Fe and Zn salts from a mixed solution comprising salts of Cu, Fe and Zn, in particular in a molar ratio Cu:Fe:Zn of 1:2-3:1-2, preferably about 1:2:1;
(i.2) drying the precipitate, preferably by heating to a temperature in a range of 50-150°C, more preferably 60-130°C, in particular about 110°C and
(i.3) calcination of the dried precipitate, preferably at a temperature in a range of 300-600°C, more preferably 350-500°C, in particular about 400°C.

In step (i.1), the mixed (aqueous) solution is preferably prepared by first adding the Fe salt, followed by the Cu salt, and then the Zn salt, in the respective molar ratios. Preferably, the respective salts are added as an aqueous solution thereof.

In step (ii) of impregnating the Cu-Fe-Zn mixed oxide particles, they are contacted with a solution of a Cs salt or Rb salt, preferably an aqueous solution thereof. Suitable salts for use in the method of the invention include Cs oxides, CsNOs, $Cs_2SO_4$, CsF, CsCl, CsBr, Csl, $Cs_3PO_4$, $Cs_2CO_3$, $Cs_2C_2O_4$, $CH_3CO_2Cs$ (with varying Cs loading, preferably from 1 to 10), and Rb oxides, RbNOs, $Rb_2SO_4$, RbF, RbCl, RbBr, Rbl, $Rb_3PO4$, $Rb_2CO_3$, $Rb_2C_2O_4$, $CH_3CO_2Rb$ (with varying Rb loading, preferably from 1 to 10), or combinations thereof. Nitrates of Cs and Rb are particularly preferred.

Step (ii) may further comprise adding one or more additional alkali elements, such as Li, Na, K or combinations thereof, preferably as an aqueous solution comprising a Li, Na, or K salt or a combination thereof. Suitable salts of these alkali elements include Li oxides, $LiNO_3$, $Li_2SO_4$, LiF, LiCl, LiBr, Lil, $Li_3PO_4$, $Li_2CO_3$, $Li_2C_2O_4$, $CH_3CO_2Li$ (with varying Li loading, preferably from 1 to 10), Na oxides, $NaNO_3$, $Na_2SO_4$, NaF, NaCl, NaBr, Nal, $Na_3PO_4$, $Na_2CO_3$, $Na_2C_2O_4$, $CH_3CO_2Na$ (with varying Na loading, preferably from 1 to 10), K oxides, $KNO_3$, $K_2SO_4$, KF, KCl, KBr, KI, $K_3PO_4$, $K_2CO_3$, $K_2C_2O_4$, $CH_3CO_2K$ (with varying K loading, preferably from 1 to 10), or combinations thereof.

A second method for preparing the hydrogenation catalyst in a one-step process comprises the steps of:

(i) providing a mixed solution comprising salts of Cu, Fe, Zn, and Cs or Rb, preferably nitrates, in particular in a molar ratio Cu:Fe:Zn of 1:2-3:1-2, preferably about 1:2:1;
(ii) inducing coprecipitation of Cs- or Rb-doped CuFeZn precipitate;
(iii) drying the precipitate, preferably by heating to a temperature in a range of 50-150°C, more preferably 60-130°C, in particular about 110°C; and
(iv) calcination of the dried precipitate, preferably at a temperature in a range of 300-600°C, more preferably 350-500°C, in particular about 400°C.

In this one-step process, salts of Cs and Rb can be selected from Cs oxides, CsNOs, $Cs_2SO_4$, CsF, CsCl, CsBr, Csl, $Cs_3PO_4$, $Cs_2CO_3$, $Cs_2C_2O_4$, $CH_3CO_2Cs$ (with varying Cs loading, preferably from 1 to 10), and Rb oxides, $RbNO_3$, $Rb_2SO_4$, RbF, RbCl, RbBr, Rbl, $Rb_3PO4$, $Rb_2CO_3$, $Rb_2C_2O_4$, $CH_3CO_2Rb$ (with varying Rb loading, preferably from 1 to 10), or combinations thereof. Nitrates of Cs and Rb are particularly preferred.

The mixed solution in step (i) may further comprise salts of one or more additional alkali elements, in particular salts of Li, Na, or K, or combinations thereof. Suitable salts of these alkali elements include Li oxides, $LiNO_3$, $Li_2SO_4$, LiF, LiCl, LiBr, Lil, $Li_3PO_4$, $Li_2CO_3$, $Li_2C_2O_4$, $CH_3CO_2Li$ (with varying Li loading, preferably from 1 to 10), Na oxides, NaNOs, $Na_2SO_4$, NaF, NaCl, NaBr, Nal, $Na_3PO_4$, $Na_2CO_3$, $Na_2C_2O_4$, $CH_3CO_2Na$ (with varying Na loading, preferably from 1 to 10), K oxides, $KNO_3$, $K_2SO_4$, KF, KCl, KBr, KI, $K_3PO_4$, $K_2CO_3$, $K_2C_2O_4$, $CH_3CO_2K$ (with varying K loading, preferably from 1 to 10), or combinations thereof.

Another aspect of the present invention relates to a method for preparing ethanol by hydrogenation of $CO_2$, comprising

(i) reacting $H_2$ and $CO_2$ in a reactor the presence of a catalyst described hereinabove; and
(ii) isolating ethanol from a reaction product obtained in (i).

The reaction can be carried out in a packed bed continuous flow reactor comprising the catalyst of the invention. $H_2$ and $CO_2$ are supplied to the reactor with a gas feed rate GHSV in a range of preferably 9000-11000 $h^{-1}$, more preferably 9500-10500 $h^{-1}$, in particular about 10000 $h^{-1}$. Accordingly, the reactor feed rate can be in a range of 0.01-0.5 g/min, preferably 0.025-0.035 g/min, more preferably about 0.03 g/min or 20-100 ml/min, preferably 50-70 ml/min, more preferably about 60 ml/min.

The reaction can be carried out at a temperature in a range of 200-400°C. Preferably, the temperature is increased during the reaction time, for example with a first period at a temperature in a range of 180-220°C, preferably for 6-10 h, increasing the temperature to a higher temperature in a range of 220-270°C and holding the temperature for a second period, preferably for 1-5 h, increasing the temperature to a higher temperature in a range of 270-350°C and holding the temperature for a third period, preferably for 3-8 h.

The pressure in the reactor can controlled to be in a range of 15-25 bar, preferably 18-22 bar, more preferably about 20 bar.

The invention will be further described by the following Figures and Examples, which are not intended to limit the scope of the invention defined by the appended claims.

**Figures**

**Fig. 1**   $N_2$ adsorption-desorption isotherms (a) and BJH pore size distribution (b) of the CuFeZn and xRb/CuFeZn catalysts.

**Fig. 2**   XRD patterns of the CuFeZn and xRb/CuFeZn catalysts.

**Fig. 3**     SEM scans of CuFeZn - (a, c) and 4%Rb/CuFeZn samples - (b, d).

**Fig. 4**     STEM-BF images of CuFeZn - (a) and 4%Rb/CuFeZn - (b) samples.

**Fig. 5**     STEM images and EDX elemental mappings of (a) CuFeZn and (b) 4%Rb/CuFeZn

**Fig. 6**     XPS spectra of (a) - survey, (b) - C 1s, (c) - O 1s, (d) - Cu 2p, (e) -Zn 2p, (f) - Fe 2p, (g) - Rb 3d in the CuFeZn and xRb/CuFeZn catalysts.

**Fig. 7**     $CO_2$ conversion - (a), STY - (b), selectivity of $C_{2+}$ alcohols and/or EtOH - (c) and MeOH selectivity - (d) over CuFeZn and xRb/CuFeZn catalysts in the gas phase. Reaction conditions: $m_{catalyst}$ = 250 mg; $H_2/CO_2$ = 3; P = 20 bar; GHSV = 9917 $h^{-1}$; TOS = 19 h; T = 200-300 °C.

**Fig. 8**     (a) - STY and (b) - product selectivity over the CuFeZn and xRb/CuFeZn catalysts; (c) - product selectivity over the 4%Rb/CuFeZn catalyst in the liquid phase. Reaction conditions: $m_{catalyst}$ = 250 mg; $H_2/CO_2$ = 3; P = 20 bar; GHSV = 9917 $h^{-1}$; TOS = 19 h; T = 200-300 °C.

**Fig. 9**     Product selectivity - (a) and STY - (b) over the 4%Rb/CuFeZn catalyst in the gas phase. Reaction conditions: $m_{catalyst}$ = 250 mg; $H_2/CO_2$ = 3; P = 20 bar; GHSV = 9917 $h^{-1}$; TOS = 19 h; T = 200-300 °C.

**Fig. 10**     (a, b) - product selectivity and STY over the 4%Rb/CuFeZn catalyst in the gas phase; (c, d) - product selectivity and STY over the 4%Rb/CuFeZn catalyst in the liquid phase. Reaction conditions: $m_{catalyst}$ = 250 mg; $H_2/CO_2$ = 3; P = 20 bar; GHSV = 9917 $h^{-1}$; TOS = 16 h; T = 300 °C.

**Fig. 11**     $N_2$ adsorption-desorption isotherms (a) and BJH pore size distribution (b) of the CuFeZn and xCs/CuFeZn catalysts.

**Fig. 12**     XRD patterns of the CuFeZn and xCs/CuFeZn catalysts.

**Fig. 13**     SEM scans of (a) - CuFeZn and (b) - 4%Cs/CuFeZn samples.

**Fig. 14**     STEM-BF images of CuFeZn - (a) and 4%Cs/CuFeZn - (b) samples.

**Fig. 15**     STEM images and EDX elemental mappings of (a) CuFeZn and (b) 4%Cs/CuFeZn samples.

**Fig. 16**     XPS spectra of (a) - survey, (b) - C 1s, (c) - O 1s, (d) - Cu 2p, (e) -Zn 2p, (f) - Fe 2p, (g) - Cs 3d in the CuFeZn and xCs/CuFeZn catalysts.

**Fig. 17**     $CO_2$ conversion - (a), STY - (b), selectivity of $C_{2+}$ alcohols or EtOH - (c) and MeOH selectivity - (d) over the CuFeZn and xCs/CuFeZn catalysts in the gas phase. Reaction conditions: $m_{catalyst}$ = 250 mg; $H_2/CO_2$ = 3; P = 20 bar; GHSV = 9917 $h^{-1}$; TOS = 19 h; T = 200-300 °C.

**Fig. 18**     (a) - STY and (b) - product selectivity over the CuFeZn and xCs/CuFeZn catalysts in the liquid phase. Reaction conditions: $m_{catalyst}$ = 250 mg; $H_2/CO_2$ = 3; P = 20 bar; GHSV = 9917 $h^{-1}$; TOS = 19 h; T = 200-300 °C.

**Fig. 19**     (a) - product distribution and (b) - STY over the 4%Cs/CuFeZn catalyst in the gas phase. Reaction conditions: $m_{catalyst}$ = 250 mg; $H_2/CO_2$ = 3; P = 20 bar; GHSV = 9917 $h^{-1}$; TOS = 19 h; T = 200-300 °C.

**Fig. 20**     (a, b) - product selectivity and STY over the 4%Cs/CuFeZn catalyst in the gas phase; (c, d) - product selectivity and STY over the 4%Cs/CuFeZn catalyst in the liquid phase. Reaction conditions: $m_{catalyst}$ = 250 mg; $H_2/CO_2$ = 3; P = 20 bar; GHSV = 9917 $h^{-1}$; TOS = 16 h; T = 300 °C.

**Fig. 21**     The reaction pathways of $CO_2$ hydrogenation over the xCs/CuFeZn catalysts.

**Examples**

**Chemicals used**

Cesium nitrate (CsNOs, 99%), rubidium nitrate (RbNOa, 99%), cupper nitrate ($Cu(NO_3)_2 \cdot 3H_2O$, 99%), zinc nitrate ($Zn(NO_3)_2 \cdot 6H_2O$, 99%), iron nitrate ($Fe(NO_3)_3 \cdot 9H_2O$, 99%), ethanol ($C_2H_5OH$, >99.8%), methanol ($CH_3OH$, >99.9%), 1-propanol ($C_3H_8O$, 99.5%), 2-propanol ($C_3H_8O$, 99.5%), 1-butanol ($C_4H_{10}O$, 99.5%), 2-butanol ($C_4H_{10}O$, 99.5%), 3-methyl-1-butanol ($C_5H_{12}O$, $\geq$98.5%), 1-pentanol ($C_5H_{12}O$, 99.5%), acetone ($C_3H_6O$, >99.5%), acetaldehyde ($C_2H_4O$, >99.5%), acetic acid ($C_2H_4O_2$, >99.7%), propanoic acid ($C_3H_6O_2$, >99%), butanoic acid ($C_4H_3O_2$, >99%), pentanoic acid ($C_5H_{10}O_2$, >99.5%), and hexanoic acid ($C_6H_{12}O_2$, >99%) were purchased from Merck.

**Example 1: Rb-loaded catalyst**

1.1 Catalyst synthesis

*Preparation of CuFeZn catalyst*

CuFeZn catalyst with a molar ratio of Cu:Fe:Zn = 1:2:1 ratio was synthesised from 0.2 M solutions from nitrate powders of $Cu(NO_3)_2$-$3H_2O$, $Fe(NO_3)_3 \cdot 9H_2O$ in $Zn(NO_3)_2 \cdot 6H_2O$. Catalyst was prepared by using a coprecipitation method. The solutions were prepared from 0.24 M $(NH_4)_2CO_3$ and were mixed at 50 °C for 30 min until completely dissolved. The solutions were then mixed together with a single-drop method while vigorously stirred and the temperature was increased to 70 °C and mixed for 1 h. After that the solution was heated to 85 °C and stirred for another 3 h. After that the solution was placed into a dryer at 110 °C overnight. After drying, the catalyst was placed into furnace for calcination at 400 °C for 2 h.

*Preparation of xRb/CuFeZn catalysts*

Catalysts composed of CuFeZn, incorporating varying Rb loadings at 2%, 4%, and 8% by weight, were synthesized through the impregnation of CuFeZn oxides in a 0.01 M aqueous solution of RbNOs. Following impregnation, the samples and the resulting solution underwent a heating process at 70 °C with vigorous stirring, subsequent overnight drying at 110 °C, and final calcination at 400 °C for 2 h.

1.2 Catalyst characterization

The textural properties of the catalysts were evaluated using the $N_2$ adsorption-desorption technique on a Micromeritics ASAP 2020 apparatus. Prior to the analysis, a 150 mg sample was degassed overnight at 200 °C (with a temperature ramp of 10 °C/min) under a vacuum of $10^{-3}$ Pa. The $N_2$ adsorption-desorption measurements were then performed at -196 °C. The pore-size distribution was determined from the desorption isotherms using the Barrett-Joyner-Halenda (BJH) method, while the total surface area was calculated using the Brunauer-Emmett-Teller (BET) method.

The composition of the solid-phase catalysts was determined using powder X-ray diffraction (XRD) analysis conducted on a PANalytical XpertPro instrument. The analysis employed CuK$\alpha$1 radiation (wavelength 1.54056 Å) over a $2\theta$ angle range from 10° to 70°, with a step size of 0.034°. The average crystallite size was estimated using the Scherrer equation, focusing on key diffraction peaks at 33.4°, 35.6°, 36.3°, 38.9°, 54.1°, and 62.9°. The relative crystallinity percentage (RC%) within the 10-70° $2\theta$ range was determined by the formula: RC% = Ic/(Ic + Ia), where Ic corresponds to the total intensity of the crystalline peaks and Ia to the total intensity of the amorphous peaks [9]. Moreover, the RC% was also calculated relative to the CuFeZn catalyst using the equation: RC% = $Ic_{(xRb/CuFeZn)}$/($Ic_{(CuFeZn)}$ + $Ia_{(CuFeZn)}$), where $Ic_{(xRb/CuFeZn)}$ represents the crystalline intensity of the Rb sample normalized against the combined intensity of the crystalline and amorphous peaks of the CuFeZn catalyst.

The elemental composition was analysed using inductively coupled plasma mass spectrometry (ICP-OES) with an Agilent 7500ce instrument. Prior to analysis, the samples were treated with a mixture of HF and $HClO_4$ to evaporate any volatiles. The remaining residue was then dissolved in a mixture of HCl and $H_3BO_4$, followed by dilution to facilitate the determination of Rb content in the catalyst samples. The morphology of the catalysts was examined using a scanning electron microscope (SEM) (FE-SEM SUPRA 35-F, Carl Zeiss) equipped with an Inca 400 energy-dispersive spectrometer (Oxford Instruments). Particle sizes for each sample were determined from the high-resolution SEM (HRSEM) images using imaged software. Additionally, scanning transmission electron microscopy (STEM) micrographs and energy-dispersive X-ray spectroscopy (EDXS) chemical mapping were performed using a JEOL ARM 200 CF microscope, which features a cold field-emission gun and a Jeol Centurio EDXS system.

The X-ray photoelectron spectroscopy (XPS) analyses were carried out on the PHI-TFA XPS spectrometer produced by Physical Electronics Inc equipped with Al-monochromatic source emitting photons at energy of 1486.6 eV. The analyzed area was 0.4 mm in diameter. Quantification of surface composition was performed from XPS peak intensities taking into account relative sensitivity factors provided by instrument manufacturer. Surface sensitivity was 2-5 nm. All data were corrected by the binding energy of C 1s (284.8 eV) adventitious carbon. Accuracy of binding energy was ± 0.3 eV [10].

**Results:**

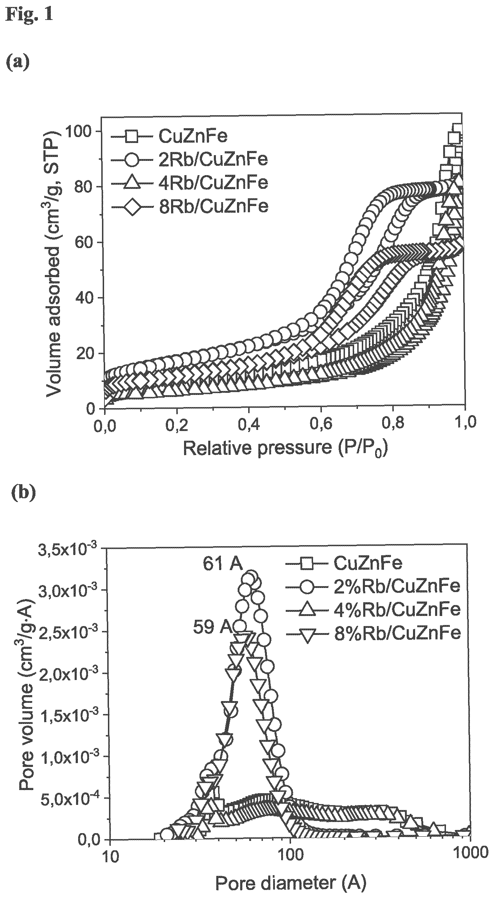

**Fig. 1** shows $N_2$ adsorption-desorption isotherms (a) and BJH pore size distribution (b) of the CuFeZn and xRb/CuFeZn catalysts.

**Table 1**

Elemental composition and structural properties of the CuFeZn and xRb/CuFeZn catalysts

| Catalyst | Rb[a] (wt %) | Cu[a] (wt %) | Zn[a] (wt %) | Fe[a] (wt %) | $S_{BET}$[b] (m²/ g) | $S_{micro}$[b] (m²/ g) | $S_{meso}$[b] (m²/ g) | $V_{micro}$[c] (cm³/g) | $V_{total}$[c] (cm³/ g) | $V_{meso}$[c] (cm³/ g) | PD[d] (nm) | Crystallite size[e] (nm) | RC[e] (%) | RC vs CuFeZn[e] (%) |
|---|---|---|---|---|---|---|---|---|---|---|---|---|---|---|
| **CuFeZn** | - | 19.0 | 21.0 | 41.0 | 31.7 | 1.9 | 29.8 | $7.6 \cdot 10^{-4}$ | 0.149 | 0.148 | 15.5 | 20.6 | 96.5 | 96.5 |
| 2%Rb/ **CuFeZn** | 2.0 | 13.0 | 21.0 | 43.0 | 60.0 | 3.8 | 56.2 | $1.6 \cdot 10^{-3}$ | 0.123 | 0.121 | 6.1 | 18.4 | 79.5 | 60.2 |
| 4%Rb/ **CuFeZn** | 3.0 | 18.0 | 19.0 | 38.0 | 23.0 | 4.3 | 18.7 | $2.1 \cdot 10^{-3}$ | 0.119 | 0.197 | 16.6 | 21.3 | 95.9 | 99.6 |
| 8%Rb/ **CuFeZn** | 6.0 | 12.0 | 20.0 | 39.0 | 41.2 | 4.7 | 36.5 | $2.2 \cdot 10^{-3}$ | 0.088 | 0.086 | 5.9 | 19.8 | 76.1 | 54.5 |

[a]ICP-OES. [b]BET method. [c]t-plot method. [d]Average pore diameter measured from the desorption branch according to the BJH method. [e]The crystallite size and relative crystallinity (RC) were obtained from XRD data.

Detecting Rb accurately with ICP-OES can be challenging, which could introduce a margin of error in the results. Additionally, there is a possibility that the analyzed portion of the sample may not have been perfectly homogeneous, further contributing to the variation in the measured amounts. However, in SEM-EDX analysis, the results showed a Rb content much closer to the theoretical values, indicating a better correlation with the amounts used during synthesis.

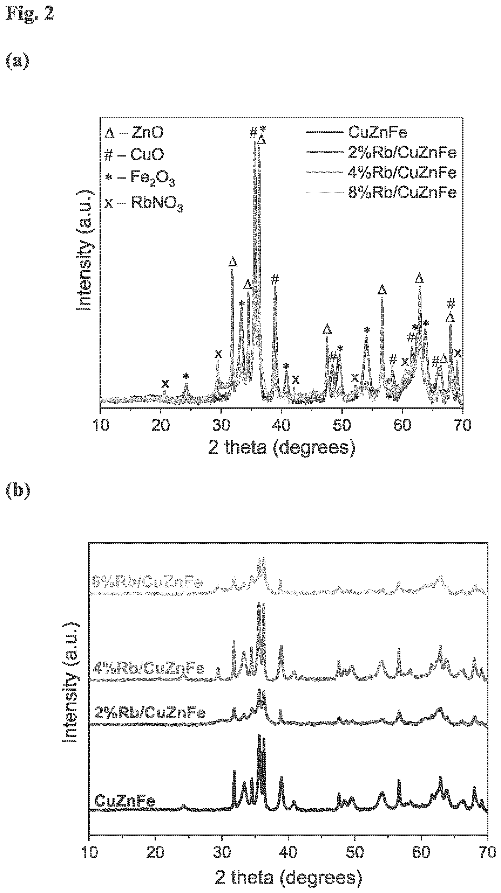

The XRD patterns displayed in Fig. 2 exhibit the profiles of the CuFeZn and xRb/CuFeZn catalysts. The primary crystalline constituents for the catalysts prior to the reaction encompass ZnO (COD No. 96-900-4182) with peaks at $2\theta$ angles of 31.8°, 34.5°, 36.3°, 47.6°, 56.7°, 62.9°, 66.4°, and 68.0°; CuO (COD No. 96-901-6327) with peaks at $2\theta$ angles of 35.6°, 38.9°, 48.4°, 58.4°, 61.7°, 66.4°, and 68.0°; $Fe_2O_3$ (COD No. 96-154-6384) with peaks at $2\theta$ angles of 24.2°, 33.3°, 35.6°, 40.9°, 49.6°, 54.1°, 62.5°, and 63.9°; as well as $RbNO_3$ (COD No. 96-210-7327) with peaks at $2\theta$ values of 20.7°, 29.5°, 42.2°, 52.3°, 61.3°, 65.4°, and 69.4°.

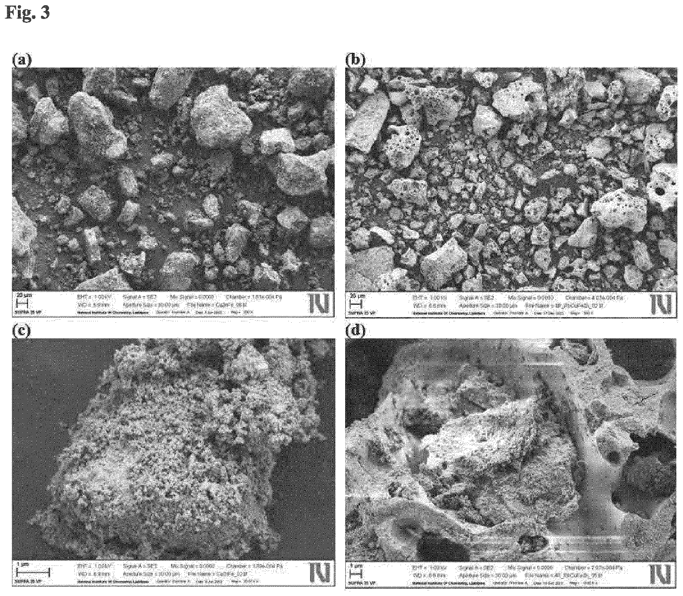

**Fig. 3** shows SEM scans of CuFeZn mixed oxides (a, c) and Rb loaded samples 4%Rb/CuFeZn (b, d).

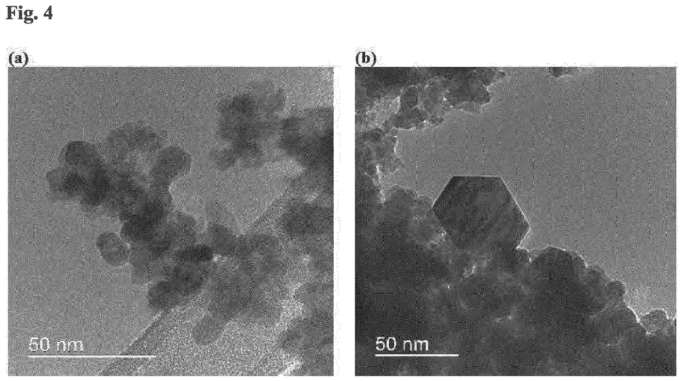

**Fig. 4** shows STEM-BF images of CuFeZn mixed oxide particles (a) and Rb-loaded samples 4%Rb/CuFeZn - (b).

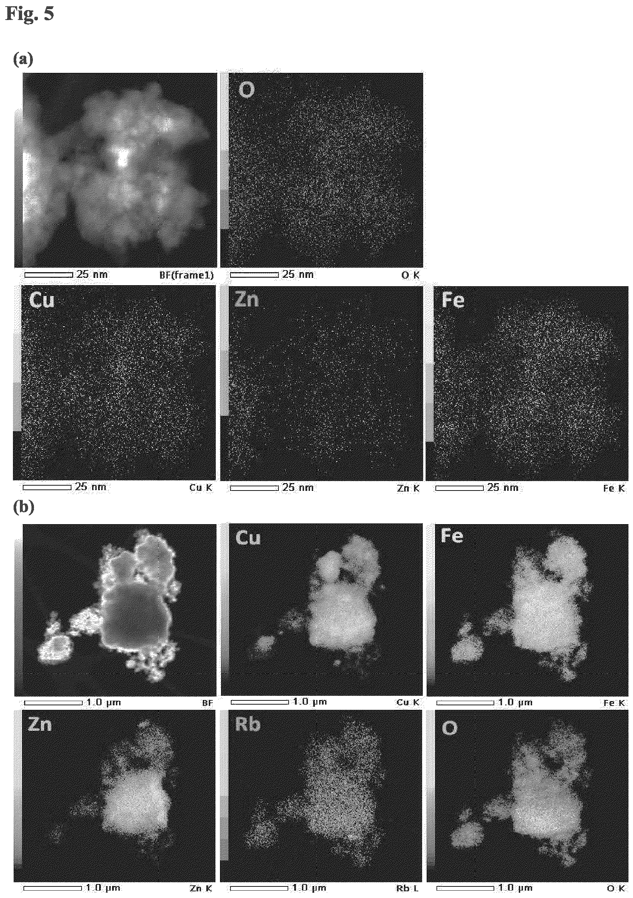

**Fig. 5** shows STEM images and EDX elemental mappings of CuFeZn mixed oxide particles (a) and Rb-loaded samples 4%Rb/CuFeZn - (b).

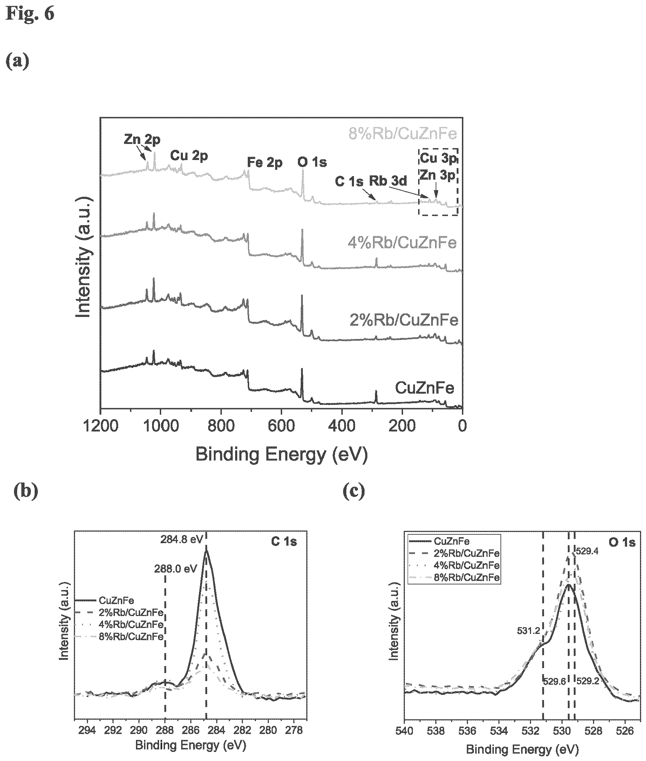

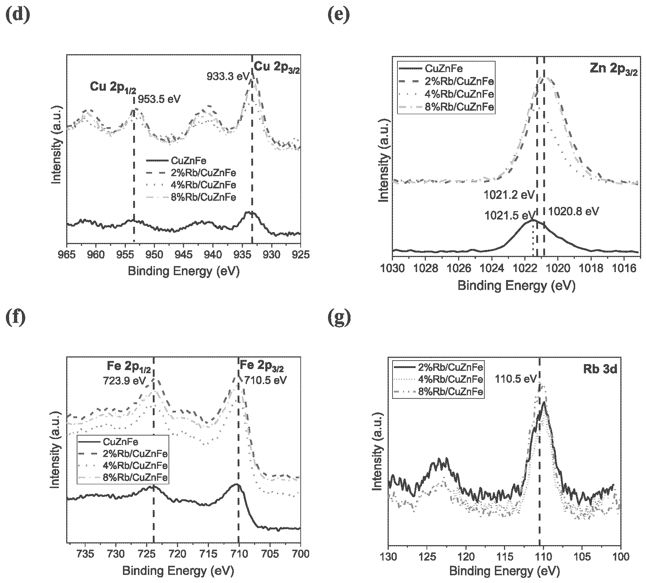

**Fig. 6** shows XPS spectra of (a) - survey, (b) - C 1s, (c) - O 1s, (d) - Cu 2p, (e) -Zn 2p, (f) - Fe 2p, (g) - Rb 3d in the CuFeZn and xRb/CuFeZn catalysts.

**Table 2**

| SEM-EDX analysis of the CuFeZn and xRb/CuFeZn catalysts. | | | |
|---|---|---|---|
| **CuFeZn** | | | |
| **Element** | **Average (wt%)** | **Scan 1 (wt%)** | **Scan 2 (wt%)** | **Scan 3 (wt%)** |
| Cu | 20.1 | 20.5 | 20.0 | 19.7 |
| Fe | 35.6 | 35.8 | 35.7 | 35.4 |
| O | 29.7 | 28.9 | 29.7 | 30.5 |
| Zn | 14.6 | 14.8 | 14.6 | 14.4 |
| **2%Rb/CuFeZn** | | | |
| Rb | 2.3 | 2.2 | 2.1 | 2.4 |
| Cu | 15.3 | 16.0 | 15.4 | 14.7 |
| Fe | 40.6 | 40.2 | 41.3 | 40.4 |
| O | 21.0 | 20.5 | 20.8 | 21.8 |
| Zn | 20.8 | 21.1 | 20.5 | 20.7 |
| **4%Rb/CuFeZn** | | | |
| Rb | 1.9 | 2.5 | 1.5 | 1.6 |
| Cu | 20.5 | 20.4 | 20.7 | 20.4 |
| Fe | 36.9 | 36.4 | 37.2 | 37.0 |
| O | 26.1 | 25.8 | 26.1 | 26.5 |
| Zn | 14.6 | 14.9 | 14.4 | 14.5 |
| **8%Rb/CuFeZn** | | | |
| Rb | 4.4 | 4.4 | 4.5 | 4.3 |
| Cu | 13.4 | 13.2 | 13.6 | 13.4 |
| Fe | 38.8 | 38.1 | 38.8 | 39.4 |
| O | 23.9 | 24.4 | 23.7 | 23.8 |
| Zn | 19.5 | 19.9 | 19.5 | 19.2 |

**Table 3**

| Surface composition of CuFeZn and xRb/CuFeZn catalysts as determined by XPS analysis. | | | | | | |
|---|---|---|---|---|---|---|
| **Catalyst** | **C 1s, at.%** | **O 1s, at.%** | **Fe 2p, at.%** | **Cu 2p, at. %** | **Zn 2p, at.%** | **Rb 3d, at.%** |
| CuFeZn | 37.4 | 44.5 | 8.2 | 5.0 | 4.8 | 0.0 |
| 2%Rb/CuFeZn | 13.2 | 56.0 | 10.2 | 9.8 | 8.6 | 2.2 |
| 4%Rb/CuFeZn | 28.4 | 51.0 | 8.8 | 4.8 | 5.1 | 1.8 |
| 8%Rb/CuFeZn | 10.2 | 58.2 | 10.2 | 97 | 8.5 | 3.3 |

1.3 Catalytic performance

The catalysts were assessed in their powdered form (0.25 g), loaded into a parallel packed bed reactor unit with quartz wool, utilizing a 6.35 mm tube. A gas chromatograph equipped with a TCD detector and CP-Molsieve and PoraPlot U column was connected to the gas outlet of the reactor. The gases employed for testing and reduction were hydrogen ($H_2$) with a purity of 5.0 (Messer) and carbon dioxide ($CO_2$) with a purity of 4.5 (Messer), mixed in a ratio of $H_2/CO_2 = 3$. Prior to the reaction, the catalyst was subjected to reduction in hydrogen gas at atmospheric pressure and 300 °C for 1 h. The gas reaction mixture was supplied through hydrogen and carbon dioxide cylinders, mixed before entering the reactor. The gas feed rate was maintained at GHSV = 9917 $h^{-1}$, and the reactor feed rate was set at (0.03 g/min or 60 ml/min), while the reaction was carried out at a pressure of 20 bar. The temperature program for the catalytic testing in the reactor consisted of holding the temperature at 200 °C for 8 h, increasing it to 250 °C for 3 h, and then raising it further to 300 °C for 5 h, with 30 min of heating between temperature ramps.

Subsequently, the liquid products obtained were analysed offline using a gas chromatography-mass spectrometry system, specifically the Agilent GC-7890A combined with the Agilent 5977B GC/MSD. This system was equipped with a DB-WAX Ultra Inert capillary column, measuring 30 m in length, having an internal diameter of 0.25 mm, and featuring a film thickness of 0.25 $\mu$m. The identification and quantification of these liquid products were achieved through an external calibration technique, spanning the 0.01-5 wt% range and employing the chemicals detailed in the chemical section.

The primary liquid products identified included EtOH, 1-propanol, 1-pentanol, 1-butanol, and MeOH. The GC-MS analysis also detected traces of liquid byproducts, including acetone, 2-butanol, 3-methyl-1-butanol, propanoic acid, butanoic acid, acetic acid, pentanoic acid, hexanoic acid, and acetaldehyde. The relative errors in the measured product concentrations were determined to be less than 5%. The carbon balance consistently exceeded 95%.

*Calculation details of catalytic performances*

The total conversion of $CO_2$ ($X_{CO2}$), was calculated using the next equation:

$$X_{CO2}(mol\%) = \frac{n(CO2)_{in} - n(CO2)_{out}}{n(CO2)_{in}} \times 100\% \tag{1}$$

where $n(CO_2)_{in}$ and $n(CO_2)_{out}$ are the input and output moles of $CO_2$ before and after the reaction, respectively.
The carbon product selectivity, $S(C_i)$, (excluding CO fraction) was calculated using the equation below [11,12]:

$$S(C_i)(mol\%) = \frac{mol(P_i) \times C_n}{\sum mol(P_i) \times C_n - n(CO)out} \times 100\% \tag{2}$$

$n(P_i)$ and $C_n$ are specified reaction product and carbon number, respectively. The values in this article are in mol percent.
While CO fraction was calculated accordingly [13]:

$$S_{CO}(mol\%) = \frac{n(CO)_{out}}{\sum mol(P_i) \times C_n} \times 100\% \tag{3}$$

The total mass balance was always higher than 98%. The space time yield (STY) was calculated as following equation (3-4), where the $n_i$ and $m_i$ represent the number moles or mass of product (EtOH and/or $C_{2+}$ alcohols), $m_{cat}$ represents the mass of catalysts (g), t represents the reaction time (h).

$$\text{STY } (\text{mmol} \cdot \text{g}_{\text{cat}}^{-1} \cdot \text{h}^{-1}) = \frac{n_i}{(m_{\text{cat}} \cdot t)} \cdot 1000 \qquad \qquad (4)$$

Gas hourly space velocity (GHSV) were calculated as follow, where FR is the volumetric flow rate of the gas mixture of $H_2$ and $CO_2$ at STP, ml/h; and $V_{cat}$ is the volume of the catalyst bed, ml.

$$\text{GHSV}_{\text{total}} (\text{h}^{-1}) = \frac{FR\left(\frac{mL}{h}\right)}{V_{\text{cat}}(mL)} \qquad \qquad (5)$$

The carbon balance (CB) was calculated according to the following:

$$CB = \frac{n(CO2)_{\text{out}} + n(CO)_{\text{out}} + \sum \text{mol}(P_i) \times C_n}{n(CO2)_{\text{in}}} \qquad \qquad (6)$$

**Results:**

*The effect of Rb loading, ZSM-5 support, reaction time and temperature on the composition of the gas product*

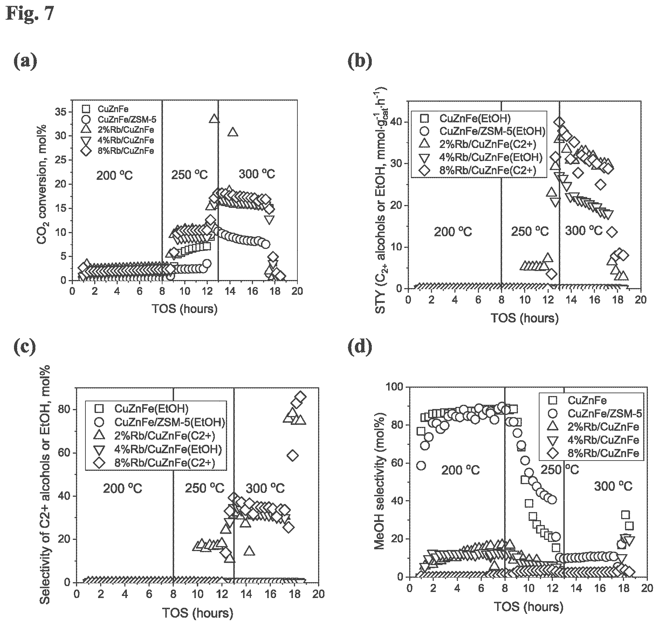

**Fig. 7** displays $CO_2$ conversion - (a), STY - (b), selectivity of C2+ alcohols and/or EtOH - (c) and MeOH selectivity - (d) over CuFeZn and xRb/CuFeZn catalysts in the gas phase.
Reaction conditions: $m_{catalyst}$ = 250 mg; $H_2/CO_2$ = 3; P = 20 bar; GHSV = 9917 h$^{-1}$; TOS = 19 h; T = 200-300 °C.

*The effect of Rb loading on the composition of the liquid product and STY*

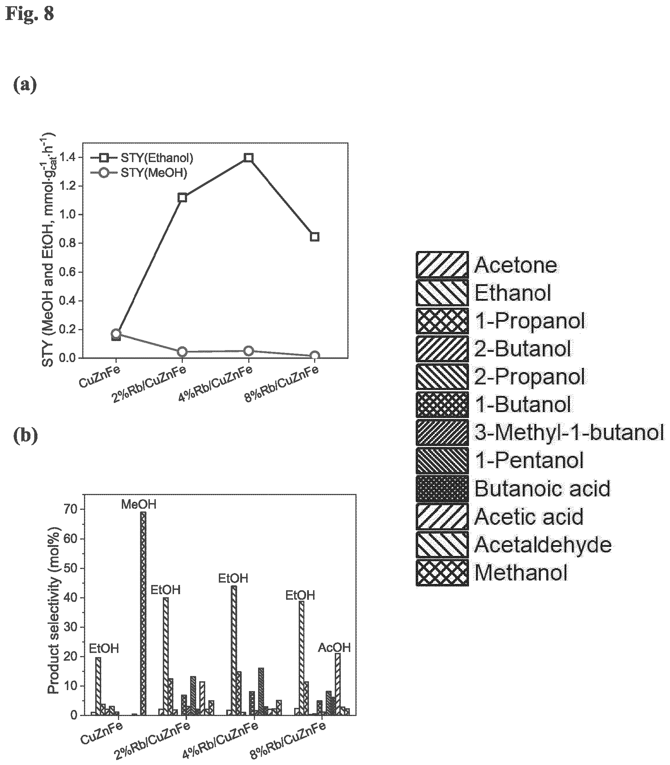

**Fig. 8** shows (a) - STY and (b) - product selectivity over the CuFeZn and xRb/CuFeZn catalysts; (c) - product selectivity over the 4%Rb/CuFeZn catalyst in the liquid phase. Reaction conditions: $m_{catalyst}$ = 250 mg; $H_2/CO_2$ = 3; P = 20 bar; GHSV = 9917 h$^{-1}$; TOS = 19 h; T = 200-300 °C.

*The product distribution over 4%Rb/CuFeZn catalyst at different reaction temperature in the gas phase*

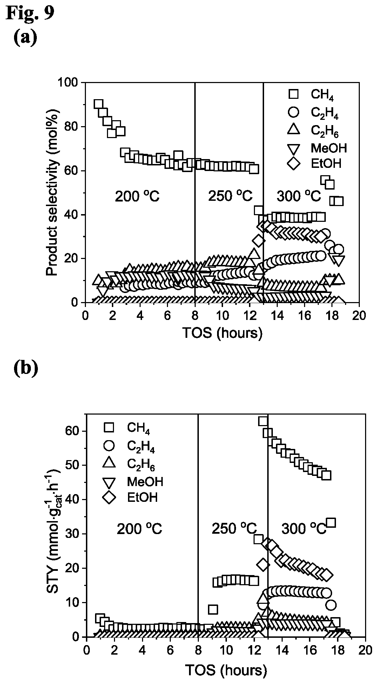

**Fig. 9** shows product selectivity - (a) and STY - (b) over the 4%Rb/CuFeZn catalyst in the gas phase. Reaction conditions: $m_{catalyst}$ = 250 mg; $H_2/CO_2$ = 3; P = 20 bar; GHSV = 9917 h$^{-1}$; TOS = 19 h; T = 200-300 °C.

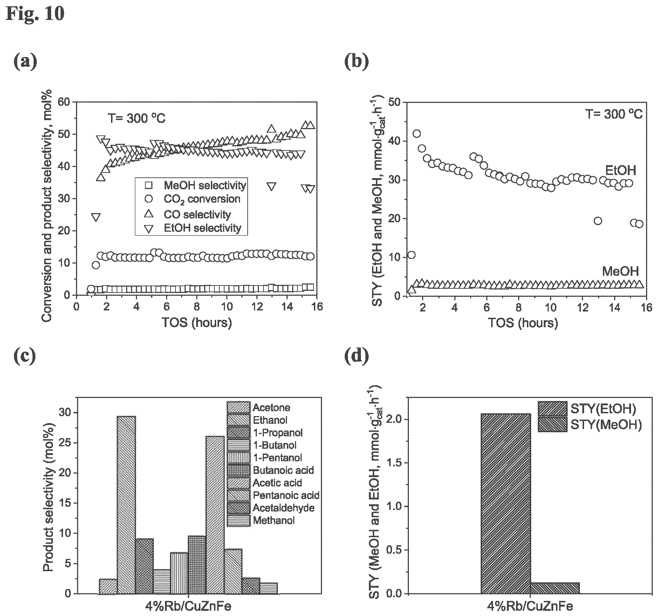

**Fig. 10** shows (a, b) - product selectivity and STY over the 4%Rb/CuFeZn catalyst in the gas phase; (c, d) - product selectivity and STY over the 4%Rb/CuFeZn catalyst in the liquid phase. Reaction conditions: $m_{catalyst}$ = 250 mg; $H_2/CO_2$ = 3; P = 20 bar; GHSV = 9917 h$^{-1}$; TOS = 16 h; T = 300 °C.

**Table 4**

The catalytic performance and reaction conditions of heterogenous Cu-based catalysts in $CO_2$ thermocatalytic hydrogenation to EtOH and/or $C_{2+}$OH in a continuous fixed-bed reactor.

| | Catalytic performance | | | | Reaction conditions | | | | |
| Catalyst | $STY_{EtOH}$ or $C_{2+}OH$ mmol·g$_{cat}$$^{-1}$·h$^{-1}$ | $X_{CO2}$ (%) | $S_{EtOH}$ or $C_{2+}OH$(%) | T (°C) | P (bar) | GHSV (h$^{-1}$) | $H_2/CO_2$ | TOS (h) | Ref. |
|---|---|---|---|---|---|---|---|---|---|
| Cu@Na-Beta | 8.64 | 14.0 | 100 | 300 | 21 | 12000 | 3 | 100 | [13] |
| CuZnAl/K-CuMgZnFe | 2.24 | 42.3 | 90.0 | 320 | 50 | N.A. | 3 | 60 | [14] |
| Cs-Cu$_{0.8}$Fe$_{1.0}$Zn$_{1.0}$ | 1.47 | 36.6 | 19.8 | 330 | 50 | N.A. | 3 | 30 | [7] |
| 4.6K-CuMgZnFe | 1.47 | 30.4 | 15.7 | 320 | 50 | N.A. | 3 | N.A. | [15] |
| Cu-CoGa-0.4 | 1.35 | 17.8 | 23.8 | 220 | 30 | 6000 mL/g/h | 3 | 3 | [16] |
| CuFeZn-0.7PDA | 1.26 | 10.6 | N.A. | 310 | 40 | 7200 | 3 | 96 | [8] |
| CuFeZn$_{0.5}$K$_{0.15}$ | 2.84 mmol/mL | 32.4 | 11.8 | 350 | 60 | 5000 | 3 | N.A. | [17] |
| Cu | 0.002 | N.A. | 84.0 | 190 | 1 | 9000 | 1 | N.A. | [18] |
| K/Cu-Zn-Fe | 290 g/L$_{cat}$/h | 44.0 | 20.0 | 300 | 70 | 5000 | 3 | 500 | [19] |

(continued)

| Catalyst | Catalytic performance | | | | | Reaction conditions | | | |
|---|---|---|---|---|---|---|---|---|---|
| | $STY_{EtOH}$ or $C_{2+}OH$ mmol·$g_{cat}^{-1}$·$h^{-1}$ | $X_{CO2}$ (%) | $S_{EtOH}$ or $C_2$ +OH(%) | T (°C) | P (bar) | GHSV ($h^{-1}$) | $H_2/CO_2$ | TOS (h) | Ref. |
| CuNaFe | 153 mg/gcat/h | 32.3 | 10 | 310 | 30 | 28800 mL/g/h | 3 | 38 | [20] |
| 0.6S-KCuFeZn | 50.7 mg/$g_{cat}$/h | 36.1 | 22.9 | | | 3000 mL/g/h | | | |
| 0.6S-KCuFeZn/ CuZnAlZr | 173.9 mg/$g_{cat}$/h | 36.6 | 18.2 | 320 | 50 | 12000 mL/g/h | 3 | 35 | [21] |
| **4%Rb/25%Cu -50%Fe-25%Zn** | **42.0** | **12.5** | **48.9** | **300** | **20** | **9917** | **3** | **19** | **This study** |
| **4%Cs/25%Cu-50% Fe-25%Zn** | **47.5** | **17.0** | **78.8** | **200-300** | **20** | **9917** | **3** | **19** | **This study** |

N.A. means that the data is not available in the reference.

## Example 2: Cs-loaded catalyst

2.1 Catalyst synthesis

2-Steps Catalyst Preparation Method:

*Preparation of CuFeZn catalyst*

Mixed CuFeZn oxide catalyst with a molar ratio of Cu:Fe:Zn = 1:2:1 ratio was prepared by using a coprecipitation method. In detail, the CuFeZn precipitates were obtained by a precipitation of Cu, Zn and Fe nitrate solution (0.2 mol/L) using aqueous $(NH_4)_2CO_3$ (0.24 mol/L) as the precipitating agent with a single-drop method at 70 °C and pH = 9 under stirring. The molar ratio of Cu:Zn:Fe was set at 1:1:2. The products were collected by centrifugation and washed with deionized water for several times, followed by drying at 80 °C for 12 h. After calcination at 400 °C for 2 h, the CuFeZn ternary metal oxides were obtained. Before commencing the reaction, all catalysts underwent a reduction process in a flow of hydrogen for a duration of 1 h.

*Preparation* of *xCs/CuFeZn*

To synthesize x%Cs/CuFeZn, the procedure involved mixing powdered CuFeZn catalyst with $CsNO_3$ salt. The $CsNO_3$ salt was dissolved in deionized water to create a 0.2 M solution, which was then heated to 70 °C and combined with the powdered catalyst. After 2 h, the mixture was left to dry overnight at 110 °C. Following the drying process, the catalyst was subjected to calcination in a furnace at 400 °C for a duration of 2 h.

1-Step Catalyst Preparation Method:

The catalyst preparation followed a 1-step method involving the dissolution of $Fe(NO_3)_3$, $Cu(NO_3)_2$, $Zn(NO_3)_2$, and CsNOs in deionized water to prepare 0.2 mol/L solutions of each metal nitrate. These solutions were mixed sequentially, starting with $Fe(NO_3)_3$, followed by $Cu(NO_3)_2$, $Zn(NO_3)_2$, and finally $CsNO_3$, ensuring the final mixture maintained a Cu:Zn:Fe molar ratio of 1:1:2. An aqueous $(NH_4)_2CO_3$ solution (0.24 mol/L) was then added dropwise to this mixed metal nitrate solution at 70 °C, with constant stirring and a maintained pH of 9, to induce coprecipitation of Cs-doped CuFeZn precipitates. The precipitates were collected by centrifugation and washed multiple times with deionized water to remove impurities. The washed precipitates were dried at 80 °C for 12 h and subsequently calcined at 400 °C for 2 h to form Cs-doped CuFeZn ternary metal oxides. Prior to catalytic testing, the calcined oxides were reduced by exposure to hydrogen gas at a specified temperature for 1 h to obtain the active reduced catalyst.

2.2 Catalyst characterization

Textural properties of the catalysts were measured by the $N_2$ adsorption-desorption method on a Micromeritics ASAP 2020 instrument. Before the experiment, the sample (150 mg) was degassed at 200 °C overnight (temperature ramp 10 °C/min) under vacuum ($10^{-3}$ Pa), and afterward, an $N_2$ adsorption-desorption analysis was carried out at -196 °C, while the pore-size distribution was calculated based on the desorption isotherms by the Barrett-Joyner-Halenda (BJH) method and the total surface area was calculated by the Brunauer-Emmett-Teller (BET) method.

The solid phase catalysts composition was identified by powder X-ray diffraction analysis on PANalytical XpertPro instrument using CuK$\alpha$1 radiation (1.54056 A) in the range of 2 theta angles ranging from 10° to 70° with increments of 0.034°. The average crystallite size was calculated using the Scherrer equation, based on the most significant peaks at 33.4°, 35.6°, 36.3°, 38.9°, 54.1°, and 62.9°. The relative crystallinity percentage (RC%) within the $2\theta$ range of 10-70° was calculated using the formula: $RC\% = Ic/(Ic + Ia)$, where $Ic$ represents the total intensity of the crystalline peaks, and Ia represents the total intensity of the amorphous peaks [9]. Additionally, the RC% was calculated relative to the CuFeZn catalyst using the following equation: $RC\% = ICc_{(xCs/CuFeZn)}(Ic_{(CuFeZn)}+a(Ic_{(CuFeZn)})$, where $Ic_{(xCs/CuFeZn)}$ is the crystalline intensity of the Cs sample divided by the total intensity of the crystalline and amorphous peaks of the CuFeZn catalyst.

The element content was determined by inductively coupled plasma mass spectroscopy (ICP-OES) using an Agilent 7500ce. Before the analysis, the samples were fumed with a mixture of HF and $HClO_4$. The residue was dissolved in the mixture of HCl and $H_3BO_4$ and diluted for determination of the Cs content in catalyst samples. The amount of carbon in the studied spent catalysts was quantified using TGA-FTIR (thermogravimetric analysis-infrared spectrometry) Spectrum 3 with EGA 4000 from PerkinElmer. The analysis of the spent catalysts was performed in the temperature range from 40 to 750 °C with a heating rate of 10 °C/min in an air stream with a flow rate of 100 ml/min.

A scanning electron microscope (SEM) (FE-SEM SUPRA 35-F, Carl Zeiss) equipped with an energy-dispersive spectrometer Inca 400 (Oxford Instruments) was used to record the morphology of the studied catalysts, while the particle size of each sample was calculated from the obtained HRSEM images using the imaged software. Scanning transmission electron microscopy (STEM) micrographs and energy dispersive X-ray spectroscopy (EDXS) chemical mapping of the samples were obtained using a JEOL ARM 200 CF microscope with a cold field-emission gun and Jeol Centurio EDXS system. More detailed experimental procedures for STEM-EDX analysis have been described in our previous work [10].

The X-ray photoelectron spectroscopy (XPS) analyses were conducted using the PHI-TFA XPS spectrometer manufactured by Physical Electronics Inc, equipped with an Al-monochromatic source emitting photons at an energy level of 1486.6 eV. The examined area had a diameter of 0.4 mm. Quantification of surface composition was determined from XPS peak intensities, taking into consideration the relative sensitivity factors provided by the instrument manufacturer. The surface sensitivity was in the range of 2-5 nm. All data underwent correction based on the binding energy of C 1s (284.8 eV) adventitious carbon. The accuracy of binding energy measurement was within $\pm$ 0.3 eV [10].

## Results:

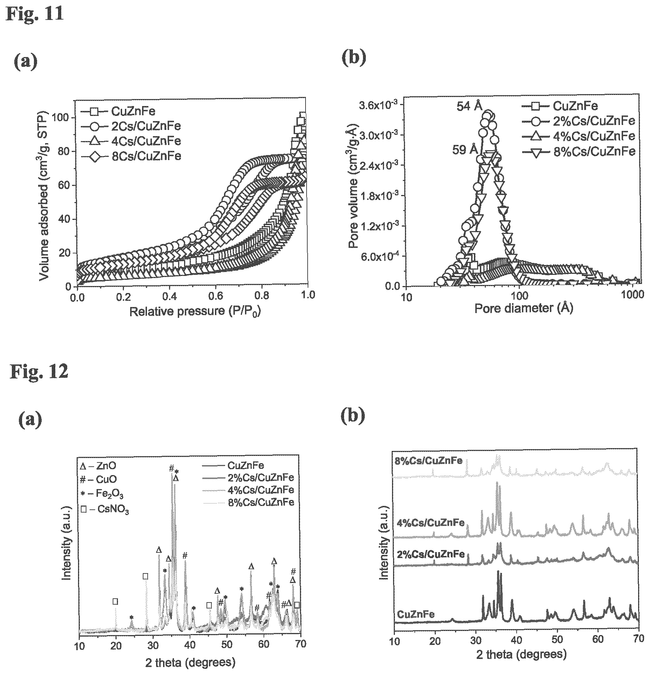

**Fig. 11** shows the $N_2$ adsorption-desorption curves and pore size distribution. The $N_2$ adsorption-desorption isotherms for the CuFeZn and xCs/CuFeZn catalysts display typical type IV isotherms with type-H3 hysteresis loops, indicating the presence of mesoporous structures. The volume of nitrogen adsorbed varies significantly among the different catalysts. The CuFeZn catalyst shows the lowest volume of $N_2$ adsorbed, suggesting a relatively lower surface area and pore volume compared to the Cs-doped samples. The 2%Cs/CuFeZn catalyst exhibits a significant increase in adsorption volume, indicating a higher surface area and pore volume, suggesting that the incorporation of 2%Cs enhances the porosity of the catalyst. The 4%Cs/CuFeZn catalyst shows a lower adsorption volume similar to the base CuFeZn catalyst, implying that this level of Cs doping does not significantly alter the surface area or porosity. The 8%Cs/CuFeZn catalyst sees an increase in adsorption volume, but not as dramatically as with the 2%Cs/CuFeZn sample, indicating an intermediate effect of Cs doping at this concentration. The BJH pore size distribution curves further elucidate the mesoporous nature of the catalysts and the impact of Cs doping. The CuFeZn catalyst displays a peak pore diameter around 35 Å with broad pore size distributions, suggesting a range of pore sizes contributing to the overall porosity. The 2% Cs/CuFeZn catalyst shows a shift in the pore size distribution with a peak pore diameter around 54 Å, and the distribution is narrower, indicating more uniform pore sizes compared to the base catalyst. The 4%Cs/CuFeZn catalyst's pore size distribution is similar to the base catalyst, with peak diameters around 35 Å, and it also has a broad pore size distribution. The 8%Cs/CuFeZn catalyst exhibits an increase in peak pore diameter to around 59 Å, similar to the 4%Cs/CuFeZn sample, with the distribution remaining narrow, suggesting a consistent mesoporous structure with increased pore sizes at higher Cs content.

The elemental composition and structural properties of the CuFeZn and xCs/CuFeZn catalysts provided in **Table 5** offer additional insights. The surface area ($S_{BET}$) and mesoporous surface area ($S_{meso}$) increase significantly with the addition of Cs, particularly in the 2%Cs/ sample, which has the highest $S_{BET}$ of 60.9 $m^2$/g and $S_{meso}$ of 59.0 $m^2$/g. This indicates that even a small amount of Cs significantly enhances the surface area. However, a further increase in Cs content to 4% and 8% results in a reduction of $S_{BET}$ and $S_{meso}$. The pore volumes ($V_{total}$ and $V_{meso}$) show a similar trend. The CuFeZn catalyst has $V_{total}$ of 0.149 $cm^3$/g and $V_{meso}$ of 0.148 $cm^3$/g. The 2%Cs/CuFeZn sample has slightly lower $V_{total}$ and $V_{meso}$ values of 0.117 $cm^3$/g and 0.116 $cm^3$/g, respectively. The 4%Cs/CuFeZn catalyst show increases in $V_{total}$ and

$V_{meso}$, indicating that higher Cs content increases the overall pore volume. However, the 8%Cs/CuFeZn sample has the lowest $V_{total}$ (0.096 cm$^3$/g) and $V_{meso}$ (0.094 cm$^3$/g) compared to the other samples, suggesting that an excess of CsNO$_3$ leads to a reduction in pore volume. The average pore diameter (PD) measured by the BJH method reveals that the CuFeZn catalyst has a PD of 15.5 nm, while the 2%Cs/CuFeZn catalyst has a significantly smaller PD of 5.4 nm. The 4% Cs/CuFeZn catalyst shows a larger PD of 19.2 nm, and the 8%Cs/CuFeZn catalyst has a PD of 5.9 nm. This variation in PD with different Cs content indicates that Cs doping not only affects the surface area and pore volume but also the pore size distribution, with the 4%Cs/CuFeZn catalyst exhibiting the largest pores.

**Table 5.** Elemental composition and structural properties of the CuFeZn and xCs/CuFeZn catalysts.

| Catalyst | Cs[a] (wt %) | Cu[a] (wt %) | Zn[a] (wt %) | Fe[a] (wt %) | $S_{BET}$[b] (m²/ g) | $S_{micro}$[b] (m²/ g) | $S_{meso}$[b] (m²/ g) | $V_{micro}$[c] (cm³ /g) | $V_{total}$ (cm³ /g) | $V_{meso}$[c] (cm³ /g) | PD[d] (nm) | Crystallite size[e] (nm) | R C[e] (% ) | RC vs CuFe Zn[e] (%) |
|---|---|---|---|---|---|---|---|---|---|---|---|---|---|---|
| CuFeZn | - | 19 | 21 | 41 | 31.7 | 1.9 | 29.8 | $7.6 \cdot 10^{-4}$ | 0.14 9 | 0.14 8 | 15. 5 | 20.6 | 96 .5 | 96.5 |
| 2%Cs/ **CuFeZn** | 2.0 | 13 | 21 | 43 | 60.9 | 1.9 | 59.0 | $4.3 \cdot 10^{-4}$ | 0.11 7 | 0.11 6 | 5.4 | 19.0 | 87 .9 | 64.3 |
| 4%Cs/ **CuFeZn** | 3.0 | 18 | 19 | 39 | 20.5 | 4.4 | 16.1 | $2.2 \cdot 10^{-3}$ | 0.12 6 | 0.12 4 | 19. 2 | 22.0 | 97 .1 | 108.6 |
| 8%Cs/Cu FeZn | 6.0 | 12 | 20 | 40 | 46.3 | 4.8 | 41.5 | $2.2 \cdot 10^{-3}$ | 0.09 6 | 0.09 4 | 5.9 | 18.1 | 91 .4 | 67.2 |

[a]ICP-OES. [b]BET method. [c]t-plot method. [d]Average pore diameter measured from the desorption branch according to the BJH method. [e]The crystallite size and relative crystallinity (RC) were obtained and calculated from XRD data.

Detecting Cs accurately with ICP-OES can be challenging, which could introduce a margin of error in the results. Additionally, there is a possibility that the analyzed portion of the sample may not have been perfectly homogeneous, further contributing to the variation in the measured amounts. However, in SEM-EDX analysis, the results showed a Cs content much closer to the theoretical values, indicating a better correlation with the amounts used during synthesis.

The XRD patterns for the synthesized samples of CuFeZn, 2%Cs/CuFeZn, 4%Cs/CuFeZn, and 8%Cs/CuFeZn reveal distinct crystalline phases, including ZnO, CuO, $Fe_2O_3$, and CsNOs **(Fig. 12)**. The presence of ZnO is confirmed by peaks at $2\theta$ values of 31.8°, 34.5°, 36.3°, 47.6°, 56.7°, 62.9°, 66.4°, and 68.0° (COD No. 96-900-4182), while CuO peaks are observed at 35.6°, 38.9°, 48.4°, 58.4°, 61.7°, 66.4°, and 68.0° (COD No. 96-901-6327). $Fe_2O_3$ is identified by peaks at 24.2°, 33.3°, 35.6°, 40.9°, 49.6°, 54.1°, 62.5°, and 63.9° (COD No. 96-154-6384), and $CsNO_3$ by peaks at 19.9°, 28.3°, and 45.5° (COD No. 96-200-1972). The structural and compositional analysis of the CuFeZn and xCs/CuFeZn catalysts, as summarized in **Table 5,** reveals significant insights into the influence of Cs loading on the crystallite size and relative crystallinity (RC) of the catalyst components. The crystallite size and RC values were derived from XRD data, providing a direct correlation between the Cs content and the structural properties of the catalysts. The base CuFeZn catalyst exhibited well-defined peaks corresponding to ZnO, CuO, and $Fe_2O_3$, indicating a relatively crystalline structure without the presence of CsNOs. This is reflected in the crystallite size of 20.6 nm and high RC values (96.5%), which suggest a stable and ordered crystalline phase. Upon introducing Cs into the CuFeZn system, significant changes in both crystallite size and RC were observed. The 2%Cs/CuFeZn catalyst shows a slight reduction in crystallite size to 19.0 nm, accompanied by a decrease in the RC (64.3%) of CuO, ZnO, and $Fe_2O_3$, particularly in comparison to the CuFeZn catalyst. This suggests that even a small amount of Cs leads to structural alterations, possibly due to the partial incorporation of Cs into the metal oxide matrix, disrupting the crystalline order. The 4%Cs/CuFeZn catalyst demonstrates an increase in crystallite size to 22.0 nm and a significant rise in the RC values, surpassing those of the base CuFeZn catalyst. This increase is particularly notable in the CuO, ZnO, and $Fe_2O_3$ phases, which suggests that Cs not only enhances the crystallization of these oxides but also promotes the formation of more ordered crystalline structures. The appearance of more intense CsNOs peaks in the XRD pattern supports this, indicating that Cs acts as a flux, aiding in the growth of well-defined crystalline domains. At the highest Cs loading of 8%, the crystallite size decreases to 18.1 nm, accompanied by a broadening of the diffraction peaks for CuO, ZnO, and $Fe_2O_3$, as well as more prominent CsNOs peaks. This broadening indicates increased lattice strain or reduced crystallite size, likely due to the further incorporation of Cs into the metal oxide lattice, disrupting the crystalline order. The reduction in RC for the metal oxides compared to the 4% Cs/CuFeZn sample further corroborates this disruption, suggesting that beyond a certain threshold, Cs incorporation leads to a deterioration in the crystalline structure. These observations underscore the dual role of Cs in modifying the structural properties of the CuFeZn catalyst. At moderate Cs levels (4%), the enhancement in crystallinity and crystallite size suggests improved structural integrity, which enhanced catalytic performance. However, at higher Cs levels (8%), the disruption of crystallinity affected catalytic activity adversely, indicating an optimal Cs loading around 4% for achieving the best balance between crystallite size, crystallinity, and catalytic performance.

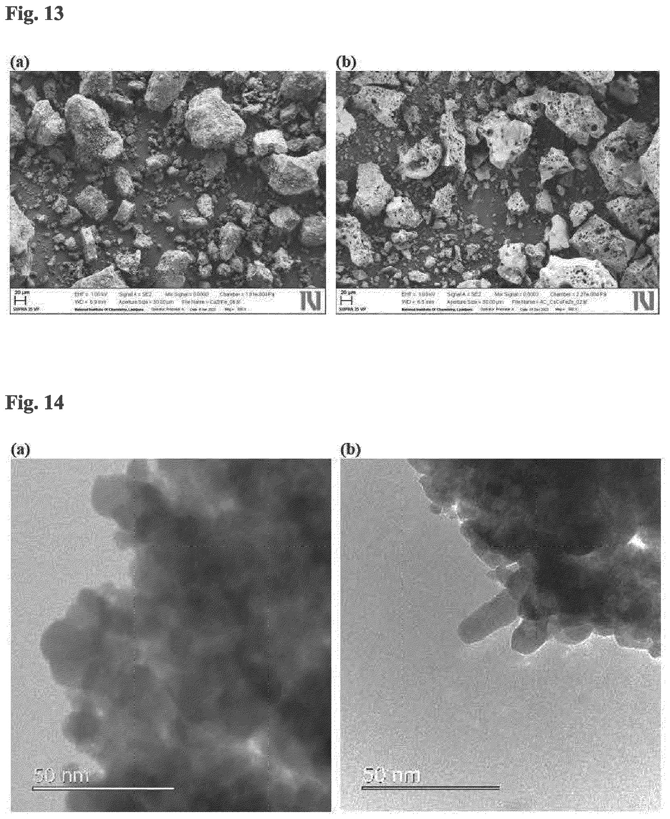

The SEM images present morphological comparisons between the CuFeZn catalyst and the 4%Cs/CuFeZn catalyst **(Fig. 13).** The CuFeZn catalyst exhibits a relatively uniform distribution of particles with a range of sizes. The particles appear to be somewhat aggregated, forming larger clusters with irregular shapes. The surface texture is rough, indicating a relatively high degree of surface area which is beneficial for catalytic activity. The 4%Cs/CuFeZn catalyst shows **(Fig. 13b)** significant morphological changes compared to the CuFeZn catalyst. The particles are more porous and less aggregated, with a distinct sponge-like structure. This indicates that the incorporation of Cs has led to the formation of a more open and porous structure. The increased porosity is beneficial as it can enhance the accessibility of reactants to the active sites of the catalyst, potentially improving catalytic performance. Additionally, the surface of the particles appears smoother compared to the CuFeZn catalyst, which might influence the interaction with reactants.

**Table 6** provides the SEM-EDX analysis data for the CuFeZn and xCs/CuFeZn catalysts, showing the elemental composition in terms of weight percentage (wt%). The SEM-EDX analysis reveals the compositional changes that occur with the incorporation of Cs into the CuFeZn catalyst matrix. For the CuFeZn catalyst, the composition remains consistent across different scans, indicating a homogeneous distribution of elements. The high Fe content (35.6 wt%) and significant amounts of Cu (20.1 wt%), O (29.7 wt%), and Zn (14.6 wt%) suggest the formation of a stable catalyst matrix. In the 2% Cs/CuFeZn catalyst, the presence of Cs at an average of 4.0 wt% introduces noticeable changes. The Cu content decreases to 13.7 wt%, while Fe increases to 39.1 wt%, and Zn to 20.2 wt%. The decrease in O content to 23.1 wt% indicates that Cs incorporation affects the oxygenated species in the catalyst. The variations in Cs content across scans (3.5 to 4.7 wt%) suggest that Cs distribution may be slightly heterogeneous at this doping level. The 4%Cs/CuFeZn catalyst shows an average Cs content of 3.8 wt%, similar to the 2% sample, but with slight variations in the distribution. The Cu content returns to around 20.1 wt%, close to the undoped sample, while Fe and O contents are 34.6 wt% and 27.2 wt%, respectively. The Zn content decreases slightly to 14.4 wt%. These changes suggest that the addition of Cs at this level leads to a redistribution of the elements within the catalyst, potentially impacting the catalyst's structural and functional properties. The 8%Cs/CuFeZn catalyst exhibits the highest Cs content at 8.7 wt%, with Cu content decreasing further to 13.3 wt%. Fe remains consistent at 34.6 wt%, while O and Zn contents are 23.5 wt% and 20.0 wt%, respectively. The increased Cs content significantly impacts the elemental composition, with variations across different scans indicating a more heterogeneous distribution of Cs. This high level of Cs incorporation likely induces substantial structural changes in the catalyst matrix, which could influence its catalytic behavior and performance. The elemental compositions from SEM-EDX, combined with the structural properties from BET and BJH analyses, provide a comprehensive understanding of how Cs incorporation affects the CuFeZn catalysts. The changes in surface area, pore volume, and pore size distribution correlate with the variations in elemental composition, highlighting the intricate relationship between composition, structure, and catalytic properties. This detailed analysis aids in optimizing the catalyst design for specific applications by adjusting the Cs content to achieve desired structural and functional characteristics.

**Table 6**

| SEM-EDX analysis of the CuFeZn and xCs/CuFeZn catalysts. | | | | |
|---|---|---|---|---|
| **CuFeZn** | | | | |
| Element | Average (wt%) | Scan 1 (wt%) | Scan 2 (wt%) | Scan 3 (wt%) |
| Cu | 20.1 | 20.5 | 20.0 | 19.7 |
| Fe | 35.6 | 35.8 | 35.7 | 35.4 |
| O | 29.7 | 28.9 | 29.7 | 30.5 |
| Zn | 14.6 | 14.8 | 14.6 | 14.4 |
| **2%Cs/CuFeZn** | | | | |
| Cs | 4.0 | 3.5 | 3.9 | 4.7 |
| Cu | 13.7 | 13.6 | 13.4 | 14.0 |
| Fe | 39.1 | 40.0 | 39.5 | 37.7 |
| O | 23.1 | 23.3 | 23.7 | 22.3 |
| Zn | 20.2 | 19.6 | 19.5 | 21.4 |
| **4%Cs/CuFeZn** | | | | |
| Cs | 3.8 | 3.5 | 4.1 | 3.9 |
| Cu | 20.1 | 20.5 | 19.6 | 20.1 |
| Fe | 34.6 | 35.0 | 34.0 | 34.7 |
| O | 27.2 | 26.5 | 28.1 | 27.0 |
| Zn | 14.4 | 14.5 | 14.2 | 14.3 |
| **8%Cs/CuFeZn** | | | | |
| Cs | 8.7 | 8.9 | 7.9 | 9.1 |
| Cu | 13.3 | 14.7 | 12.5 | 12.5 |
| Fe | 34.6 | 34.8 | 33.3 | 35.8 |
| O | 23.5 | 23.2 | 23.6 | 23.7 |
| Zn | 20.0 | 18.4 | 22.7 | 18.8 |

**Fig. 14a** and **Fig. 14b** present the scanning transmission electron microscopy bright-field (STEM-BF) images of the CuFeZn and 4%Cs/CuFeZn catalysts, respectively. These images provide insights into the morphology and structural characteristics of the catalysts at the nanoscale. In **Fig. 14a,** the STEM-BF image of the CuFeZn sample reveals a heterogeneous distribution of particles with varying sizes. The particles appear to be agglomerated, forming clusters with indistinct boundaries. The average particle size is difficult to ascertain precisely due to the agglomeration, but it appears to be in the range of 10-30 nm. The introduction of 4%Cs appears to influence the particle morphology slightly **(Fig. 14b).**

The particles in this sample are more uniformly dispersed compared to the CuFeZn sample, with clearer boundaries and a more defined structure. The average particle size seems to be more consistent, also in the range of 10-30 nm, but with better dispersion and less agglomeration. The presence of Cs likely contributes to this improved dispersion, which can enhance the catalytic performance by providing more accessible active sites and reducing diffusion limitations. The STEM-BF images of the CuFeZn and 4%Cs/CuFeZn catalysts reveal significant differences in their morphological characteristics. The improved dispersion and reduced agglomeration in the 4%Cs/CuFeZn sample suggest that the addition of Cs enhances the structural properties of the catalyst, potentially leading to better catalytic activity and selectivity in $CO_2$ hydrogenation reactions.

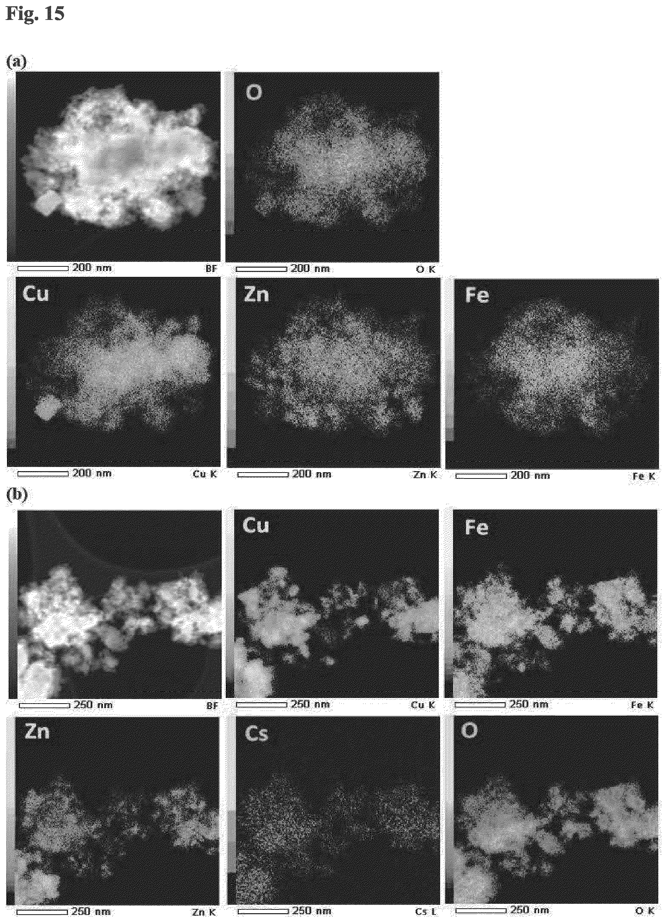

**Fig. 15a** and **Fig. 15b** present the scanning transmission electron microscopy (STEM) images and energy-dispersive X-ray (EDX) elemental mappings of the CuFeZn and 4%Cs/CuFeZn catalysts, respectively. These analyses provide valuable information on the elemental distribution and homogeneity within the catalyst samples. In **Fig. 15a,** the STEM image of the CuFeZn sample, coupled with the EDX mappings, shows the distribution of O, Cu, Zn, and Fe within the catalyst. The EDX mappings indicate a relatively uniform distribution of these elements throughout the sample. The Cu, Zn, and Fe signals are well-dispersed, suggesting a homogeneous composition. The presence of oxygen is also uniform, indicating that the metal oxides are evenly distributed. The addition of 4%Cs is clearly evident in the EDX mapping **(Fig. 15b).** The elemental distribution of Cu, Zn, Fe, and O remains relatively uniform, similar to the CuFeZn sample. However, the Cs signal shows a distinct distribution, indicating successful incorporation of Cs into the catalyst structure. The presence of Cs appears to be well-integrated with the other elements, suggesting that the addition of Cs does not disrupt the overall homogeneity of the catalyst but rather enhances it. These observations are significant for understanding the catalytic performance. The homogeneous distribution of elements suggests that the active sites are uniformly available, which is beneficial for catalytic reactions. The incorporation of Cs enhances the structural properties without disrupting the uniformity, potentially leading to improved catalytic activity and selectivity in $CO_2$ hydrogenation reaction into EtOH. The successful integration of Cs could also contribute to the observed improvements in dispersion and reduced agglomeration noted in the structural characterization, further supporting the catalyst's enhanced performance.

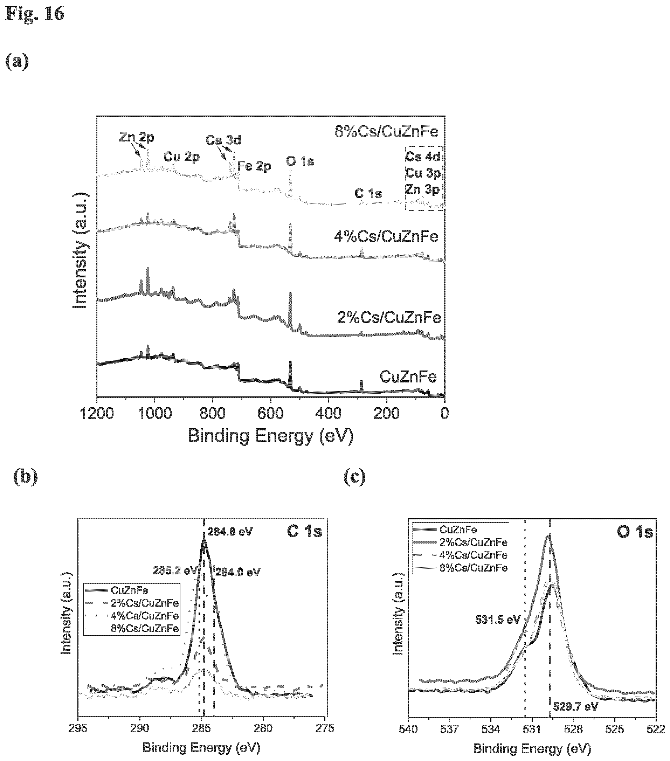

The XPS survey spectra presented in **Fig. 16a** provide a comprehensive overview of the elemental composition of the CuFeZn and xCs/CuFeZn catalysts, where x represents different loadings of Cs. The survey spectra indicate the presence of core-level peaks corresponding to C 1s, O 1s, Fe 2p, Cu 2p, Zn 2p, and Cs 3d in all samples. The presence of these peaks confirms that the CuFeZn catalysts have been successfully synthesized and that the Cs has been incorporated into the catalysts as intended. The XPS spectra of the C 1s region **(Fig. 16b)** for the CuFeZn, 2%Cs/CuFeZn, 4%Cs/CuFeZn, and 8%Cs/CuFeZn samples exhibit significant differences, indicating the impact of Cs addition on the surface chemistry of the catalyst. For the undoped CuFeZn sample and the samples doped with 2% and 8% Cs, the C 1s spectra are dominated by a peak centered at approximately 284.8 eV. This binding energy is typically associated with carbon in a hydrocarbon-like environment (C-C or C-H bonds). The consistent presence of this peak across these samples suggests that the primary carbon environment remains largely unchanged with the addition of Cs in low (2%) and high (8%) concentrations. In contrast, the 4%Cs/CuFeZn sample exhibits two distinct peaks at 284.0 eV and 285.2 eV. The peak at 284.0 eV is generally attributed to C-C or C-H bonds, which could be linked to adventitious carbon or carbonaceous residues on the catalyst surface. The peak at 285.2 eV, however, indicates the presence of oxygenated carbon species, such as C-O bonds, which could be due to the interaction of the catalyst surface with oxygen-containing groups or intermediates. The appearance of these two distinct peaks only in the 4%Cs/CuFeZn sample suggests that this particular Cs concentration optimally alters the surface chemistry, enhancing the formation or stability of oxygenated species that could be critical for catalytic activity. The intensity of the C 1s peaks decreases upon the addition of Cs to the CuFeZn catalyst, indicating a reduction in the amount of detectable carbon species on the surface as Cs content increases. This decrease in intensity is most pronounced in the 8%Cs/CuFeZn sample, suggesting that high Cs loading may lead to significant surface coverage or passivation, which reduces the exposure of carbon species to the X-ray beam during analysis. Interestingly, the 4%Cs/CuFeZn sample shows the least reduction in peak intensity compared to the undoped CuFeZn sample, despite the shift and splitting of peaks. This implies that while Cs modifies the surface, at this specific concentration, it does so in a way that preserves or even enhances certain reactive carbon species, potentially leading to better catalytic performance.

**Fig. 16c** presents the O 1s spectra with two main peaks centered at 529.7 eV and 531.5 eV. The peak at 529.7 eV is typically assigned to lattice oxygen within the metal(s) oxide structure, while the peak at 531.5 eV corresponds to surface hydroxyl groups or adsorbed oxygen species. The Cs-modified catalysts show a slight shift and increased intensity in the higher binding energy component, suggesting an enhanced interaction between oxygen species and the catalyst surface, which may influence catalytic performance. Interestingly, a shoulder at 531.5 eV is observed only in the undoped CuFeZn and 4%Cs/CuFeZn samples. This shoulder is indicative of a higher concentration of surface hydroxyl groups or adsorbed oxygen species, which are critical for catalytic reactions involving oxygen. The presence of this shoulder in the 4% Cs/CuFeZn sample suggests that this particular Cs concentration optimizes the interaction between the catalyst surface and oxygen species, potentially enhancing catalytic activity. In contrast, the 2%Cs/CuFeZn and 8%Cs/CuFeZn samples do not exhibit this shoulder, indicating a reduced presence of surface oxygen species. This absence might be due to insufficient surface modification at low Cs concentrations (2%) or excessive surface passivation at high Cs concentrations (8%), which can decrease the availability of active oxygen species necessary for catalytic processes.

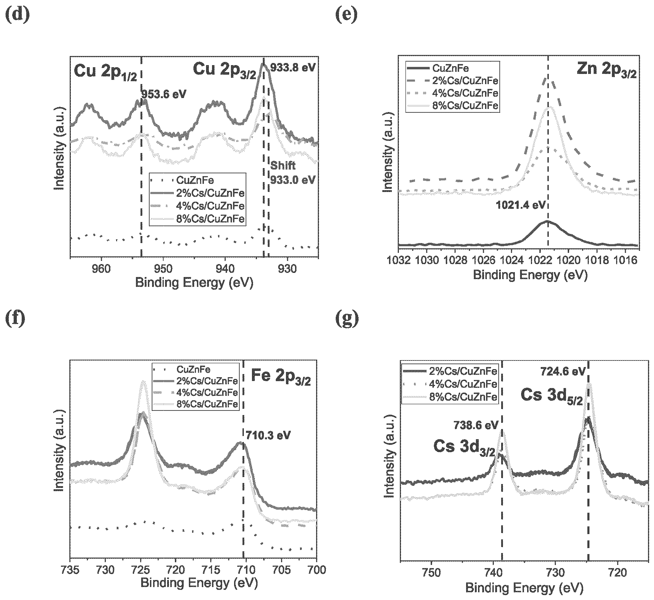

The Cu 2p spectra, shown in **Fig. 16d,** feature two main peaks corresponding to Cu $2p_{3/2}$ and Cu $2p_{1/2}$ at binding energies of 933.8 eV and 953.6 eV, respectively. The presence of satellite peaks (at about 942 eV and 962 eV) suggests the coexistence of $Cu^{2+}$ species [8]. For the 4%Cs/CuFeZn sample, the Cu $2p_{3/2}$ peak shifts slightly to a lower binding energy, from 933.8 eV to 933.0 eV. This shift suggests a change in the electronic environment surrounding the Cu ions, likely due to an interaction between Cu and Cs atoms. The shift to lower binding energy can be interpreted as a decrease in the oxidation state of Cu or a redistribution of electron density around Cu ions, potentially caused by electron donation from Cs or changes in the local coordination environment. Interestingly, this shift is most pronounced in the 4%Cs/CuFeZn sample, which correlates with the previously observed trends in the O 1s and C 1s spectra. This consistency across different XPS spectra suggests that the 4%Cs doping level induces an optimal electronic environment that may enhance the catalytic properties of the CuFeZn catalyst.

**Fig. 16e** shows the Zn 2p spectra with a peak at 1021.4 eV, characteristic of $Zn^{2+}$ in the catalyst. The binding energy remains consistent across the different samples, suggesting that the addition of Cs does not significantly affect the electronic environment of Zn within the catalyst. The Fe 2p spectra **(Fig. 16f)** display a peak at 710.3 eV, which corresponds to $Fe^{3+}$ species. Similar to Zn, the binding energy of Fe remains largely unchanged upon Cs modification, indicating that the Fe species maintain their oxidation state and electronic environment within the catalyst matrix. Finally, the Cs 3d spectra in **Fig. 16g** show two peaks at 724.6 eV and 738.6 eV, corresponding to Cs $3d_{5/2}$ and Cs $3d_{3/2}$, respectively. These peaks confirm the successful incorporation of Cs into the catalyst structure. The intensity of these peaks increases with higher Cs loading, as expected, confirming the increasing amount of Cs on the catalyst surface.

The surface composition of the CuFeZn and xCs/CuFeZn catalysts, as revealed by XPS analysis **(Table 7),** was determined. The unmodified CuFeZn catalyst shows a balanced surface composition but lacks the beneficial modification from Cs, potentially limiting its activity. In contrast, the 4%Cs/CuFeZn catalyst exhibits a relatively high surface carbon content (35.3%), compared to the 2%Cs/CuFeZn (12.5%) and 8%Cs/CuFeZn (11.2%) catalysts. This higher carbon content may indicate the presence of surface carbonates or adventitious carbon, which could stabilize active sites or prevent excessive oxidation of metallic components. The 4%Cs/CuFeZn catalyst also has an intermediate oxygen content (46.8%), compared to the 2% (55.4%) and 8% (56.9%) Cs/CuFeZn catalysts, suggesting that this oxygen level provides an optimal balance of lattice oxygen and surface hydroxyl groups necessary for redox reactions. The slightly lower Fe 2p content in the 4%Cs/CuFeZn catalyst (7.1%) compared to the 2%Cs/CuFeZn and 8%Cs/CuFeZn catalysts (9.0% each) suggests a more finely dispersed or selectively exposed form of Fe, which could enhance catalytic activity. Despite having a lower Cu content (3.7%) than the 2%Cs/CuFeZn (10.1%) and 8%Cs/CuFeZn (8.3%) catalysts, the 4%Cs/CuFeZn catalyst's superior performance suggests that the quality or oxidation state of copper, rather than quantity, is crucial for catalytic efficiency. Additionally, the lower Zn 2p content (4.2%) in the 4%Cs/CuFeZn catalyst, compared to the 2% Cs/CuFeZn (10.6%) and 8%Cs/CuFeZn (9.3%) catalysts, indicates a more favorable distribution of Zn species, which can prevent agglomeration and maintain a high surface area for active sites. The intermediate Cs 3d content in the 4% Cs/CuFeZn catalyst (3.0%) likely offers an optimal level of surface modification, enhancing basicity and electronic properties without excessively covering active sites, leading to its superior catalytic performance.

**Table 7**

| Surface composition of CuFeZn and xCs/CuFeZn catalysts as determined by XPS analysis. | | | | | | |
|---|---|---|---|---|---|---|
| **Catalyst** | **C 1s, at.%** | **O 1s, at.%** | **Fe 2p, at.%** | **Cu 2p, at.%** | **Zn 2p, at.%** | **Cs 3d, at.%** |
| CuFeZn | 37.4 | 44.5 | 8.2 | 5.0 | 4.8 | 0.0 |
| 2%Cs/CuFeZn | 12.5 | 55.4 | 9.0 | 10.1 | 10.6 | 2.5 |
| 4%Cs/CuFeZn | 35.3 | 46.8 | 7.1 | 3.7 | 4.2 | 3.0 |
| 8%Cs/CuFeZn | 11.2 | 56.9 | 9.0 | 8.3 | 9.3 | 5.2 |

**Results:**

*The effect of* Cs *loading, reaction time and temperature on the composition of the gas products*

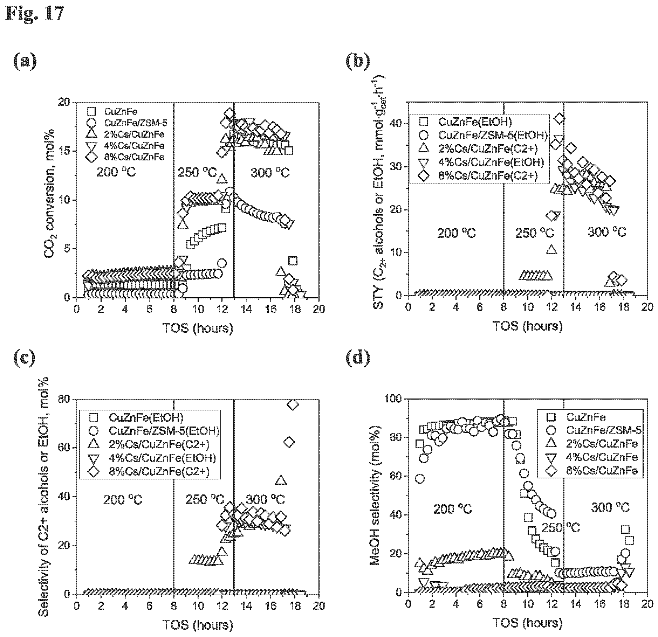

**Fig. 17** presents a comprehensive analysis of the performance of various CuFeZn and xCs/CuFeZn catalysts in the gas phase for $CO_2$ hydrogenation, with a focus on $CO_2$ conversion, STY, selectivity of $C_{2+}$ alcohols or EtOH, and MeOH selectivity. **Fig. 17a** illustrates the $CO_2$ conversion over TOS for the different catalysts at three distinct temperatures: 200, 250, and 300 °C. Initially, at 200 °C, all catalysts exhibit relatively low $CO_2$ conversion rates, with minimal differences among them. However, as the temperature increases to 250 °C and subsequently to 300 °C, a significant enhancement in $CO_2$ conversion is observed across all catalysts. Notably, the Cs-promoted catalysts, particularly the 4%Cs/CuFeZn and 8%Cs/CuFeZn, demonstrate superior performance compared to the unmodified CuFeZn catalyst. The highest $CO_2$ conversion is achieved at 300 °C, indicating that elevated temperatures favor the activation and conversion of $CO_2$ over these catalysts. In **Fig. 17b,** the STY of $C_{2+}$ alcohols and/or EtOH is depicted as a function of TOS for the catalysts at the same temperature ranges. At 200 °C, the STY values are minimal, aligning with the low $CO_2$ conversion observed. As the temperature rises to 250 °C, an increase in STY is noted, with the Cs-promoted catalysts again outperforming the unmodified CuFeZn catalyst. The trend continues at 300 °C, where the STY reaches its peak for the 4%Cs/CuFeZn and 8%Cs/CuFeZn catalysts, suggesting that Cs promotion significantly enhances the catalytic activity for the formation of higher alcohols (HA) and ethanol (EtOH). **Fig. 17c** shows the selectivity of $C_{2+}$ alcohols or EtOH in mol% over the different catalysts. At 200 °C, the selectivity remains low across all catalysts. As the temperature increases to 250 °C and further to 300 °C, there is a noticeable rise in the selectivity of $C_{2+}$ alcohols or EtOH, particularly for the Cs-modified catalysts. The 8%Cs/CuFeZn catalyst exhibits the highest selectivity at 300 °C, indicating that higher cesium loading promotes the formation of $C_{2+}$ alcohols and ethanol over methanol. **Fig. 17d** illustrates the MeOH selectivity over TOS for the catalysts. At 200 °C, the CuFeZn/ZSM-5 catalyst shows the highest methanol selectivity, maintaining a stable performance over the entire TOS. As the temperature increases to 250 °C and then to 300 °C, the methanol selectivity decreases for all catalysts, with a more pronounced decline for the Cs-promoted catalysts. This inverse relationship between methanol selectivity and temperature suggests that higher temperatures and Cs promotion favor the production of higher alcohols and ethanol over methanol.

*The effect of Cs loading on the composition of the liquid products and STY*

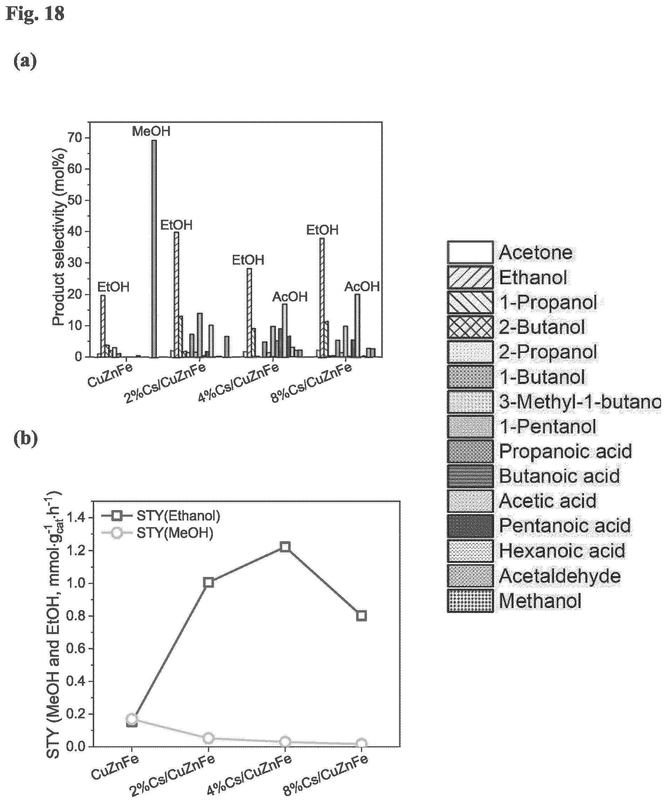

**Fig. 18** illustrates the effect of Cs loading on the STY and product selectivity over CuFeZn and Cs-loaded CuFeZn catalysts in the liquid phase. As shown in **Fig. 18a,** the introduction of Cs to the CuFeZn catalyst significantly impacts the production rates of MeOH and EtOH. The CuFeZn catalyst without Cs shows minimal STY for both methanol and ethanol. With 2%Cs loading, the STY of ethanol increases noticeably, reaching approximately 1.0 mmol·$g_{cat}$⁻¹·h⁻¹, while the STY of methanol remains low. At 4% Cs loading, ethanol STY peaks and maintains the highest value observed (-1.2 mmol·$g_{cat}$⁻¹·h⁻¹), whereas methanol STY stays relatively low and constant. However, at 8% Cs loading, the STY of ethanol drops significantly, indicating that excessive Cs may inhibit ethanol production, while methanol STY remains largely unchanged. **Fig. 18b** provides detailed insights into product selectivity over various catalysts, complementing the STY trends observed in **Fig. 18a** and highlighting the distribution of various liquid-phase products, including alcohols, acids, and other compounds. For the CuFeZn catalyst, methanol is the main product, with a selectivity of approximately 70%, alongside minor quantities of ethanol, acetone, 1-propanol, 2-butanol, 2-propanol, and 1-butanol. With the addition of 2%Cs to CuFeZn, ethanol selectivity surges to around 40%, while methanol selectivity decreases to about 10%. Additionally, minor products such as 1-propanol, 1-pentanol, and acetic acid are present in low quantities. At 4% Cs/CuFeZn, ethanol selectivity remains around 30%, indicating that this Cs loading favors ethanol production, but at the same time, more byproducts are formed and detected in the reaction mixture. In contrast, methanol selectivity drops significantly compared to the CuFeZn catalyst, with moderate amounts of acetic acid and other byproducts. At 8% Cs/CuFeZn, selectivity shifts notably towards acetic acid (-20%), with ethanol selectivity increasing to around 40%, and methanol remaining a minor product. These results suggest that Cs loading alters the catalytic pathways, promoting different products at varying Cs concentrations. Moderate Cs loading (4%) optimizes ethanol production, but shifts the selectivity towards acetic acid and other byproducts, possibly due to changes in the active sites or reaction intermediates.

*The product distribution over 4%Cs/CuFeZn catalyst at different reaction temperatures in the gas phase*

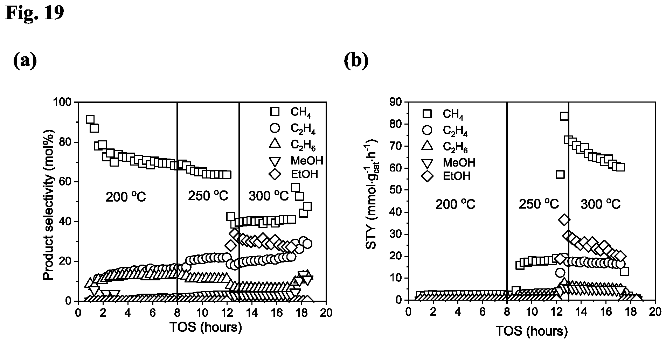

The performance of the 4%Cs/CuFeZn catalyst in the gas-phase conversion of $CO_2$ to various products was evaluated under varying temperature conditions (200-300 °C) as illustrated in **Fig. 19.** The product selectivity profile shows distinct trends for different hydrocarbons and oxygenates as the temperature increases from 200 to 300 °C. At 200 °C, $CH_4$ is the predominant product, with its selectivity starting at approximately 90% and decreasing gradually to about 70% over 8 h. $C_2H_4$ and $C_2H_5$ exhibit a slight increase in selectivity, stabilizing around 10-15% after 8 h. EtOH and methanol MeOH show minimal selectivity at this temperature. At 250 °C, the selectivity for $CH_4$ continues to decline, settling around 60%. The selectivity for EtOH increases significantly, reaching approximately 30% after 12 h. $C_2H_4$ and MeOH exhibit a minor increase in selectivity, with $C_2H_6$ stabilizing around 10%. When the temperature is further increased to 300 °C, $CH_4$ selectivity drops sharply to about 40%. Conversely, the selectivity for EtOH peaks at this temperature, keeps stable achieving nearly 30%. The selectivity for $C_2H_4$ decreases slightly but remains significant at around 20%. MeOH and $C_2H_6$ selectivity remains low and stable, with slight increase to 10% after 17 h.

**Fig. 19b** shows the STY of various products at different temperatures. At 200 °C, the STY values for all detected products were low, with $CH_4$ having the highest STY of less than 5 mmol·$g_{cat}$⁻¹·h⁻¹. When the temperature increased to 250 °C, the STY of $CH_4$ rose to 20 mmol·$g_{cat}$⁻¹·h⁻¹, while the STY of other products remained nearly unchanged. A significant change was observed at 300 °C, where the STY of EtOH increased markedly to 40 mmol·$g_{cat}$⁻¹h⁻¹. During this time, the STY of $CH_4$ initially increased to 85 mmol·$g_{cat}$⁻¹·h⁻¹ but subsequently decreased to 60 mmol·$g_{cat}$⁻¹·h⁻¹ after 18 h. Additionally, the STY for $C_2H_4$, $C_2H_6$, and MeOH increased, with $C_2H_4$ reaching around 20 mmol·$g_{cat}$⁻¹h⁻¹, and $C_2H_6$ and MeOH reaching approximately 5 mmol·$g_{cat}$⁻¹·h⁻¹ each. The data indicate that the 4%Cs/CuFeZn catalyst exhibits a strong temperature dependence in its activity and selectivity for $CO_2$ hydrogenation. At lower temperatures (200 °C), $CH_4$

is the dominant product. As the temperature increases, there is a noticeable shift towards higher selectivity and STY for ethanol and ethylene, especially at 300 °C. This suggests that higher temperatures favor the formation of more valuable $C_{2+}$ oxygenates and hydrocarbons, potentially due to enhanced activation and hydrogenation of $CO_2$ and intermediates. The ability to tune the product distribution by adjusting the reaction temperature highlights the versatility of the 4% Cs/CuFeZn catalyst in $CO_2$ hydrogenation applications.

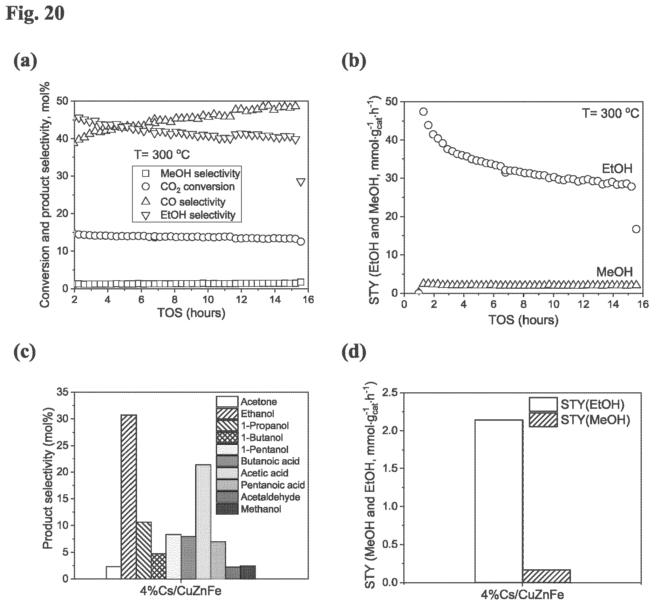

**Fig. 20a** and **Fig. 20b** illustrate the performance of the 4%Cs/CuFeZn catalyst in the gas phase. In **Fig. 20a,** the $CO_2$ conversion remains relatively stable around 15% throughout the 16 h period, indicating relatively good stability of the catalyst. EtOH selectivity decreases slightly after an initial peak, stabilizing around 40%. MeOH selectivity, on the other hand, is relatively low but stable, hovering around 2%. **Fig. 20b** focuses on the STY of EtOH and MeOH. The STY of EtOH starts high at approximately 50 mmol·$g_{cat}^{-1}$·$h^{-1}$ but gradually decreases to about 30 mmol·$g_{cat}^{-1}$·$h^{-1}$ by the end of the 16 h period. The STY of MeOH remains consistently low, around 3 mmol·$g_{cat}^{-1}$·$h^{-1}$ reflecting its lower selectivity.

**Fig. 20c-d** present the product selectivity and STY of the 4%Cs/CuFeZn catalyst in the liquid phase under identical reaction conditions. **Fig. 20c** illustrates a diverse range of products, with EtOH being the most selective, accounting for over 30% of the total product composition. Other alcohols, such as 1-propanol, 1-butanol, 1-pentanol, and methanol, each range between 2-10%. Additionally, acetic acid demonstrates significant selectivity at approximately 20%, while other detected acids, including butanoic and pentanoic acid, range between 7-8%. The presence of various alcohols and acids indicates a complex reaction network, likely involving multiple reaction pathways.

The data presented in **Fig. 20d** demonstrates a significant disparity in the STY between EtOH and MeOH. Specifically, the STY for EtOH is substantially higher than that for methanol, with ethanol achieving an STY of approximately 2.2 mmol·$g_{cat}^{-1}$·$h^{-1}$, whereas MeOH only reaches around 0.1 mmol·$g_{cat}^{-1}$·$h^{-1}$. This observation is consistent with the selectivity data, indicating a preferential formation of EtOH over MeOH by the catalyst. Furthermore, the STY of EtOH varies notably between the gas and liquid phases. In the gas phase, ethanol achieves an impressive STY of 50 mmol·$g_{cat}^{-1}$·$h^{-1}$, which is significantly higher than its STY in the liquid phase. On the other hand, the STY of MeOH remains consistently low across both phases. This further underscores the catalyst preferential activity towards EtOH production under the given experimental conditions. These findings suggest that the catalytic conditions and the nature of the catalyst employed are highly favorable for EtOH production. The stark contrast in STY between EtOH and MeOH indicates that the reaction conditions are optimized for EtOH formation.

*Reaction mechanism*

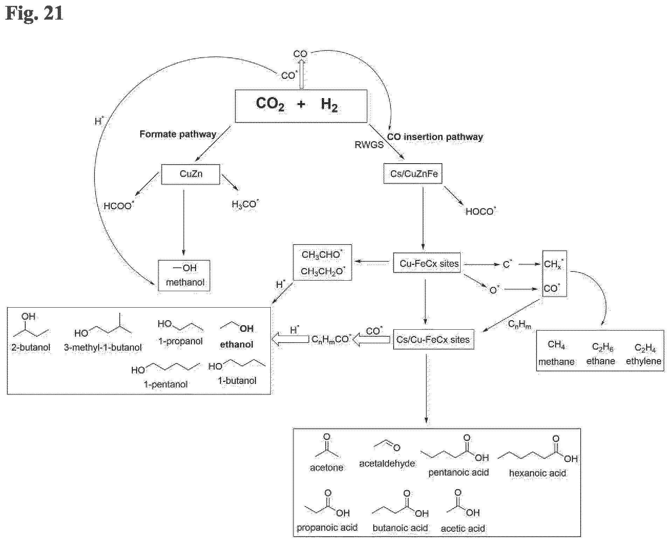

The reaction pathways of $CO_2$ hydrogenation over the xCs/CuFeZn catalysts illustrated in **Fig. 21.** The process is divided into two main pathways: the formate pathway and the CO insertion pathway. In the formate pathway, $CO_2$ and $H_2$ initially react to form formate intermediates (HCOO* and $H_3$CO*) with the aid of CuZn interfaces. These formate species are then reduced to methanol, while the presence of Cs promoter is hindered the formation of methanol. In the CO insertion pathway, the RWGS reaction converts $CO_2$ into CO and $H_2O$. This CO is further processed into formyl radicals (HOCO') using Cs/CuFeZn catalysts. These formyl radicals decompose into C* and O* at Cu-FeCx sites. The carbon radicals can subsequently form $CH_4$, $C_2H_6$, and $C_2H_4$. Meanwhile, oxygen radicals interact with carbon radicals to produce CO*. Additionally, ethanol and higher alcohols such as 1-propanol, 2-butanol, 1-pentanol, and 3-methyl-1-butanol are formed through the hydrogenation of corresponding aldehydes and ketones at Cs/Cu-FeCx sites. Furthermore, various aldehydes like acetaldehyde and acids such as acetic acid, propanoic acid, butanoic acid, pentanoic acid, and hexanoic acid are produced via further oxidation and hydrogenation steps involving CO intermediates. This comprehensive mechanism highlights the complex interactions between different catalytic steps and intermediates, leading to the formation of a wide variety of chemical compounds from $CO_2$ and $H_2$.

2.4 Conclusions

Producing ethanol through $CO_2$ hydrogenation, while achieving high selectivity and maintaining catalyst stability, poses a considerable scientific and technological challenge. An effective approach to address these issues is through innovative catalyst design. Our study focuses on a catalyst composed of Cs, Cu, Zn, and Fe, which has shown promising results in synthesizing $C_{2+}$ alcohols with high selectivity (78.8 mol%) and a $CO_2$ conversion rate of 17.0%. The catalyst, optimized to a composition of 4%Cs/25%Cu-25%Zn-50%Fe, delivers an impressive STY for ethanol of 47.5 mmol·$g_{cat}^{-1}$·$h^{-1}$ in the gas phase, significantly surpassing the performance of known catalysts in existing literature. The catalyst's large pore diameter, high pore volume, substantial crystallite size, excellent crystallinity, and abundance of basic sites primarily contribute to its high catalytic efficiency. The XPS analysis reveals that the introduction of 4%Cs into the CuFeZn catalysts modifies the electronic environment, particularly around the copper and oxygen species, which could potentially influence the catalytic properties. Our investigation into the reaction mechanism indicates that the catalyst operates via a tandem pathway, incorporating both CO insertion and the formate pathway, with the latter being the predominant route. These results underscore the potential of the xCs/CuFeZn catalyst in overcoming current challenges in

**EP 4 729 170 A1**

ethanol production through $CO_2$ hydrogenation, representing a notable advancement in the field of catalyst development.

**References**

[1] F. Zeng, C. Mebrahtu, X. Xi, L. Liao, J. Ren, J. Xie, H.J. Heeres, R. Palkovits, Catalysts design for higher alcohols synthesis by CO2 hydrogenation: Trends and future perspectives, Appl. Catal. B Environ. 291 (2021) 120073. https://doi.org/10.1016/j.apcatb.2021.120073.

[2] S. De, A. Dokania, A. Ramirez, J. Gascon, Advances in the Design of Heterogeneous Catalysts and Thermocatalytic Processes for CO2 Utilization, ACS Catal. 10 (2020) 14147-14185. https://doi.org/10.1021/acscatal.0c04273.

[3] I.C. Have, J.J.G. Kromwijk, M. Monai, F. Meirer, B.M. Weckhuysen, E.B. Sterk, Uncovering the reaction mechanism behind CoO as active phase for CO2 hydrogenation, Nat. Commun. 13 (2022). https://doi.org/10.1038/s41467-022-27981-x.

[4] A. Kostyniuk, D. Bajec, B. Likozar, One-step synthesis of ethanol from glycerol in a gas phase packed bed reactor over hierarchical alkali-treated zeolite catalyst materials, Green Chem. 22 (2020) 753-765. https://doi.org/10.1039/c9gc03262b.

[5] Y. Wang, K. Wang, B. Zhang, X. Peng, X. Gao, G. Yang, H. Hu, M. Wu, N. Tsubaki, Direct Conversion of CO2 to Ethanol Boosted by Intimacy-Sensitive Multifunctional Catalysts, ACS Catal. 11 (2021) 11742-11753. https://doi.org/10.1021/acscatal.1c01504.

[6] B. An, Z. Li, Y. Song, J. Zhang, L. Zeng, C. Wang, W. Lin, Cooperative copper centres in a metal-organic framework for selective conversion of CO2 to ethanol, Nat. Catal. 2 (2019) 709-717. https://doi.org/10.1038/s41929-019-0308-5.

[7] D. Xu, M. Ding, X. Hong, G. Liu, S.C.E. Tsang, Selective C2+ Alcohol Synthesis from Direct CO2 Hydrogenation over a Cs-Promoted Cu-Fe-Zn Catalyst, ACS Catal. 10 (2020) 5250-5260. https://doi.org/10.1021/acscatal.0c01184.

[8] C. Yang, B. Wang, Y. Wen, M. Fan, Y. Jia, S. Zhou, W. Huang, Composition control of CuFeZn catalyst derived by PDA and its effect on synthesis of C2+ alcohols from CO2, Fuel. 327 (2022) 125055. https://doi.org/10.1016/j.fuel.2022.125055.

[9] G.T. Jaya, R. Insyani, J. Park, A.F. Barus, M.G. Sibi, V. Ranaware, D. Verma, J. Kim, One-pot conversion of lignocellulosic biomass to ketones and aromatics over a multifunctional Cu-Ru/ZSM-5 catalyst, Appl. Catal. B Environ. 312 (2022) 121368. https://doi.org/10.1016/j.apcatb.2022.121368.

[10] A. Kostyniuk, D. Bajec, P. Djinović, B. Likozar, Allyl alcohol production by gas phase conversion reactions of glycerol over bifunctional hierarchical zeolite-supported bi- and trimetallic catalysts, Chem. Eng. J. 397 (2020) 125430. https://doi.org/10.1016/j.cej.2020.125430.

[11] P. Tian, G. Zhan, J. Tian, K.B. Tan, M. Guo, Y. Han, T. Fu, J. Huang, Q. Li, Direct CO2 hydrogenation to light olefins over ZnZrOx mixed with hierarchically hollow SAPO-34 with rice husk as green silicon source and template, Appl. Catal. B Environ. 315 (2022) 121572. https://doi.org/10.1016/j.apcatb.2022.121572.

[12] Y. Lou, F. Jiang, W. Zhu, L. Wang, T. Yao, S. Wang, B. Yang, B. Yang, Y. Zhu, X. Liu, CeO2 supported Pd dimers boosting CO2 hydrogenation to ethanol, Appl. Catal. B Environ. 291 (2021) 120122. https://doi.org/10.1016/j.apcatb.2021.120122.

[13] L. Ding, T. Shi, J. Gu, Y. Cui, Z. Zhang, C. Yang, T. Chen, M. Lin, P. Wang, N. Xue, L. Peng, X. Guo, Y. Zhu, Z. Chen, W. Ding, CO2 Hydrogenation to Ethanol over Cu@Na-Beta, Chem. 6 (2020) 2673-2689. https://doi.org/10.1016/j.chempr.2020.07.001.

[14] D. Xu, H. Yang, X. Hong, G. Liu, S.C. Edman Tsang, Tandem Catalysis of Direct CO2 Hydrogenation to Higher Alcohols, ACS Catal. 11 (2021) 8978-8984. https://doi.org/10.1021/acscatal.1c01610.

[15] D. Xu, M. Ding, X. Hong, G. Liu, Mechanistic aspects of the role of K promotion on Cu-Fe-based catalysts for higher alcohol synthesis from CO2 hydrogenation, ACS Catal. 10 (2020) 14516-14526. https://doi.org/10.1021/acscatal.0c03575.

[16] G. Zhang, G. Fan, L. Zheng, F. Li, Ga-Promoted CuCo-Based Catalysts for Efficient CO2Hydrogenation to Ethanol: The Key Synergistic Role of Cu-CoGaOxInterfacial Sites, ACS Appl. Mater. interfaces. 14 (2022) 35569-35580. https://doi.org/10.1021/acsami.2c07252.

[17] H. Guo, S. Li, F. Peng, H. Zhang, L. Xiong, C. Huang, C. Wang, X. Chen, Roles Investigation of Promoters in K/Cu-Zn Catalyst and Higher Alcohols Synthesis from CO2 Hydrogenation over a Novel Two-Stage Bed Catalyst Combination System, Catal. Letters. 145 (2015) 620-630. https://doi.org/10.1007/s10562-014-1446-7.

[18] S. Santanta, M.T.M. Koper, H.M. Alisson, L.H. Vieira, E.M. Assaf, M. Assaf, J.F. Gomes, Ethanol formation from CO2 hydrogenation at atmospheric pressure using Cu catalysts: Water as a key component, Appl. Catal. B, Environ. 324 (2023) 122221. https://doi.org/10.1016/j.apcatb.2022.122221.

[19] M. Takagawa, A. Okamoto, H. Fujimura, Y. Izawa, H. Arakawa, Ethanol synthesis from carbon dioxide and hydrogen, Stud. Surf. Sci. Catal. 114 (1998) 525-528. https://doi.org/10.1016/s0167-2991(98)80812-4.

[20] Z. Si, L. Wang, Y. Han, J. Yu, Q. Ge, C. Zeng, J. Sun, Synthesis of Alkene and Ethanol in CO2 Hydrogenation on a Highly Active Sputtering CuNaFe Catalyst, ACS Sustain. Chem. Eng. 10 (2022) 14972-14979. https://doi.org/10.1021/acssuschemeng.2c05450.

[21] Y. Wang, X. Zhang, X. Hong, G. Liu, Sulfate-Promoted Higher Alcohol Synthesis from CO2 Hydrogenation, ACS Sustain. Chem. Eng. 10 (2022) 8980-8987. https://doi.org/10.1021/acssuschemeng.2c02743.

**Claims**

1. A hydrogenation catalyst, comprising or essentially consisting of Cu-Fe-Zn mixed oxide particles loaded with Cs or Rb in an amount of 2-8 wt.%, based on the total weight of the loaded Cu-Fe-Zn mixed oxide particles.

2. The catalyst of claim 1, wherein Cs or Rb is loaded in an amount of 3-6 mol %, preferably 3.5-5 mol %, in particular about 4 mol %, based on the total weight of the loaded Cu-Fe-Zn mixed oxide particles.

3. The catalyst of claim 1 or 2, comprising

   Cu in an amount of 10-25 wt.%, preferably 18-22 wt.%, more preferably about 20 wt.%, Zn in an amount of 10-25 wt.%, preferably 12-16 wt.%, more preferably about 15 wt.%, and
   Fe in an amount of 33-45 wt.%, preferably 35-38 wt.%, more preferably about 37 wt.%, based on the total weight of the loaded Cu-Fe-Zn mixed oxide particles,
   in particular wherein Cu, Fe and Zn are present in a molar ratio of 1:2-3:1-2, preferably about 1:2:1.

4. The catalyst of any one of the preceding claims, wherein the catalyst is a powder comprising particles with an average particle size in a range of 10-50 nm, preferably 15-30 nm.

5. The catalyst of any one of the preceding claims, wherein the Cu-Fe-Zn mixed oxide particles loaded with Cs or Rb are porous particles comprising mesopores and micropores.

6. The catalyst of claim 5, wherein the average pore diameter is in a range of 5-20 nm, preferably 6-18 nm.

7. A method for preparing the hydrogenation catalyst of any one of claims 1-6, comprising:

   (i) providing Cu-Fe-Zn mixed oxide particles;
   (ii) impregnating the Cu-Fe-Zn mixed oxide particles with a solution of a Cs salt or Rb salt, preferably an aqueous solution of $CsNO_3$ or $RbNO_3$;
   (iii) drying the impregnated particles, preferably by heating to a temperature in a range of 50-150°C, more preferably 60-130°C, in particular about 110°C;
   (iv) calcination of the dried particles, preferably at a temperature in a range of 300-600°C, more preferably 350-500°C, in particular about 400°C.

8. The method of claim 7, wherein step (ii) further comprises adding one or more additional alkali elements, such as Li, Na, K or combinations thereof, preferably as an aqueous solution comprising a Li, Na, or K salt or a combination thereof.

9. The method of claim 7 or 8, wherein providing Cu-Fe-Zn mixed oxide particles comprises:

   (i.1) coprecipitating Cu, Fe and Zn salts from a mixed solution comprising salts of Cu, Fe and Zn, preferably nitrates, in particular in a molar ratio Cu:Fe:Zn of 1:2-3:1-2, preferably about 1:2:1;
   (i.2) drying the precipitate, preferably by heating to a temperature in a range of 50-150°C, more preferably 60-130°C, in particular about 110°C and
   (i.3) calcination of the dried precipitate, preferably at a temperature in a range of 300-600°C, more preferably 350-500°C, in particular about 400°C.

10. A method for preparing the hydrogenation catalyst of any one of claims 1-6, comprising:

    (i) providing a mixed solution comprising salts of Cu, Fe, Zn, and Cs or Rb, preferably nitrates, in particular in a molar ratio Cu:Fe:Zn of 1:2-3:1-2, preferably about 1:2:1;
(ii) inducing coprecipitation of Cs- or Rb-doped CuFeZn precipitate;
(iii) drying the precipitate, preferably by heating to a temperature in a range of 50-150°C, more preferably 60-130°C, in particular about 110°C; and
(iv) calcination of the dried precipitate, preferably at a temperature in a range of 300-600°C, more preferably 350-500°C, in particular about 400°C.

11. The method of claim 10, wherein the mixed solution in step (i) further comprises salts, in particular nitrates, of one or more additional alkali elements, in particular salts of Li, Na, or K, or combinations thereof.

12. A method for preparing ethanol by hydrogenation of $CO_2$, comprising

(i) reacting $H_2$ and $CO_2$ in a reactor the presence of a catalyst according to any one of claims 1-6; and
(ii) isolating ethanol from a reaction product obtained in (i).

13. The method of claim 12, wherein the reaction is carried out in a packed bed continuous flow reactor comprising the catalyst of any one of claims 1-6, and wherein $H_2$ and $CO_2$ are supplied to the reactor with a gas feed rate GHSV in a range of 9000-11000 $h^{-1}$, preferably 9500-10500 $h^{-1}$, more preferably about 10000 $h^{-1}$, and/or a reactor feed rate of 0.01-0.5 g/min, preferably 0.025-0.035 g/min, more preferably about 0.03 g/min or 20-100 ml/min, preferably 50-70 ml/min, more preferably about 60 ml/min.

14. The method of claim 12 or 13, wherein the reaction is carried out at a temperature in a range of 200-400°C, wherein the temperature is preferably increased during the reaction time,

in particular with a first period at a temperature in a range of 180-220°C, preferably for 6-10 h,
increasing the temperature to a higher temperature in a range of 220-270°C and holding the temperature for a second period, preferably for 1-5 h,
increasing the temperature to a higher temperature in a range of 270-350°C and holding the temperature for a third period, preferably for 3-8 h.

15. The method of any one of claims 12-14, wherein the reaction is carried out at a pressure in a range of 15-25 bar, preferably 18-22 bar, more preferably about 20 bar.

**Fig. 1**

**(a)**

**(b)**

**Fig. 2**

**(a)**

**(b)**

Fig. 3

Fig. 4

(a)

(b)

Fig. 5

(a)

(b)

**Fig. 6**

**(a)**

**(b)**

**(c)**

(d)

(e)

(f)

(g)

Fig. 7

(a)

(b)

(c)

(d)

Fig. 8

(a)

(b)

**Fig. 9**

**(a)**

**(b)**

Fig. 10

(a)

(b)

(c)

(d)

**Fig. 11**

**(a)**

**(b)**

**Fig. 12**

**(a)**

**(b)**

**Fig. 13**

**Fig. 14**

**Fig. 15**

(a)

(b)

**Fig. 16**

**(a)**

**(b)**

**(c)**

(d)

(e)

(f)

(g)

**Fig. 17**

**(a)**

**(b)**

**(c)**

**(d)**

Fig. 18

(a)

(b)

Fig. 19

(a)

(b)

**Fig. 20**

(a)

(b)

(c)

(d)

Fig. 21

Europäisches
Patentamt

European
Patent Office

Office européen
des brevets

## EUROPEAN SEARCH REPORT

**Application Number**

EP 24 20 7402

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | XU DI ET AL: "Selective C 2+ Alcohol Synthesis from Direct CO2 Hydrogenation over a Cs-Promoted Cu-Fe-Zn Catalyst", ACS CATALYSIS, vol. 10, no. 9, 9 April 2020 (2020-04-09), pages 5250-5260, XP093262378, US ISSN: 2155-5435, DOI: 10.1021/acscatal.0c01184 * abstract; page 5250 * * page 5251, right-hand column, last paragraph - page 5252, left-hand column, paragraph 1; table 1 * * page 5252, right-hand column, paragraph 2 - page 5253, right-hand column, paragraph 2; figure 2 * * figure 1 * -& XU DI ET AL: "Selective C2+ Alcohol Synthesis from Direct CO2 Hydrogenation over a Cs-Promoted Cu-Fe-Zn Catalyst - Supporting Information", ACS CATALYSIS, vol. 10, no. 9, 9 April 2020 (2020-04-09), XP093262773, DOI: doi:10.1021/acscatal.0c01184 Retrieved from the Internet: URL:https://pubs.acs.org/doi/10.1021/acscatal.0c01184#_i14> * page 2, paragraph 1 * * page 5, paragraph 2 * * figures S5,S13,S16 *    ----- -/-- | 1-15 | INV. B01J23/80 B01J37/02 B01J37/03 C07C29/156 C07C31/08 |
| | | | **TECHNICAL FIELDS SEARCHED (IPC)** B01J C07C |

The present search report has been drawn up for all claims

| Place of search | Date of completion of the search | Examiner |
|---|---|---|
| Munich | 24 March 2025 | Beckmann, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

**EP 24 20 7402**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PAMEI MALA ET AL: "Challenges and advances in understanding the roadmap for direct hydrogenation of carbon dioxide to ethanol", CATALYSIS REVIEWS: SCIENCE AND ENGINEERING, 17 September 2024 (2024-09-17), pages 1-47, XP093262458, US ISSN: 0161-4940, DOI: 10.1080/01614940.2024.2400974 * abstract; page 1 * * figure 21a; table 1 * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 24 March 2025 | Beckmann, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................................................

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **F. ZENG** ; **C. MEBRAHTU** ; **X. XI** ; **L. LIAO** ; **J. REN** ; **J. XIE** ; **H.J. HEERES** ; **R. PALKOVITS**. Catalysts design for higher alcohols synthesis by CO2 hydrogenation: Trends and future perspectives. *Appl. Catal. B Environ.*, 2021, vol. 291, 120073
- **S. DE** ; **A. DOKANIA** ; **A. RAMIREZ** ; **J. GASCON**. Advances in the Design of Heterogeneous Catalysts and Thermocatalytic Processes for CO2 Utilization. *ACS Catal.*, 2020, vol. 10, 14147-14185, https://doi.org/10.1021/acscatal.0c04273
- **I.C. HAVE** ; **J.J.G. KROMWIJK** ; **M. MONAI** ; **F. MEIRER** ; **B.M. WECKHUYSEN** ; **E.B. STERK**. Uncovering the reaction mechanism behind CoO as active phase for CO2 hydrogenation. *Nat. Commun.*, 2022, vol. 13, https://doi.org/10.1038/s41467-022-27981-x
- **A. KOSTYNIUK** ; **D. BAJEC** ; **B. LIKOZAR**. One-step synthesis of ethanol from glycerol in a gas phase packed bed reactor over hierarchical alkali-treated zeolite catalyst materials. *Green Chem.*, 2020, vol. 22, 753-765, https://doi.org/10.1039/c9gc03262b
- **Y. WANG** ; **K. WANG** ; **B. ZHANG** ; **X. PENG** ; **X. GAO** ; **G. YANG** ; **H. HU** ; **M. WU** ; **N. TSUBAKI**. Direct Conversion of CO2 to Ethanol Boosted by Intimacy-Sensitive Multifunctional Catalysts. *ACS Catal.*, 2021, vol. 11, 11742-11753, https://doi.org/10.1021/acscatal.1c01504
- **B. AN** ; **Z. LI** ; **Y. SONG** ; **J. ZHANG** ; **L. ZENG** ; **C. WANG** ; **W. LIN**. Cooperative copper centres in a metal-organic framework for selective conversion of CO2 to ethanol. *Nat. Catal.*, 2019, vol. 2, 709-717, https://doi.org/10.1038/s41929-019-0308-5
- **D. XU** ; **M. DING** ; **X. HONG** ; **G. LIU** ; **S.C.E. TSANG**. Selective C2+ Alcohol Synthesis from Direct CO2 Hydrogenation over a Cs-Promoted Cu-Fe-Zn Catalyst. *ACS Catal.*, 2020, vol. 10, 5250-5260, https://doi.org/10.1021/acscatal.0c01184
- **C. YANG** ; **B. WANG** ; **Y. WEN** ; **M. FAN** ; **Y. JIA** ; **S. ZHOU** ; **W. HUANG**. Composition control of CuFeZn catalyst derived by PDA and its effect on synthesis of C2+ alcohols from CO2. *Fuel*, 2022, vol. 327, 125055, https://doi.org/10.1016/j.fuel.2022.125055
- **G.T. JAYA** ; **R. INSYANI** ; **J. PARK** ; **A.F. BARUS** ; **M.G. SIBI** ; **V. RANAWARE** ; **D. VERMA** ; **J. KIM**. One-pot conversion of lignocellulosic biomass to ketones and aromatics over a multifunctional Cu-Ru/ZSM-5 catalyst. *Appl. Catal. B Environ.*, 2022, vol. 312, 121368, https://doi.org/10.1016/j.apcatb.2022.121368
- **P. TIAN** ; **G. ZHAN** ; **J. TIAN** ; **K.B. TAN** ; **M. GUO** ; **Y. HAN** ; **T. FU** ; **J. HUANG** ; **Q. LI**. Direct CO2 hydrogenation to light olefins over ZnZrOx mixed with hierarchically hollow SAPO-34 with rice husk as green silicon source and template. *Appl. Catal. B Environ.*, 2022, vol. 315, 121572, https://doi.org/10.1016/j.apcatb.2022.121572
- **Y. LOU** ; **F. JIANG** ; **W. ZHU** ; **L. WANG** ; **T. YAO** ; **S. WANG** ; **B. YANG** ; **B. YANG** ; **Y. ZHU** ; **X. LIU**. CeO2 supported Pd dimers boosting CO2 hydrogenation to ethanol. *Appl. Catal. B Environ.*, 2021, vol. 291, 120122, https://doi.org/10.1016/j.apcatb.2021.120122
- **L. DING** ; **T. SHI** ; **J. GU** ; **Y. CUI** ; **Z. ZHANG** ; **C. YANG** ; **T. CHEN** ; **M. LIN** ; **P. WANG** ; **N. XUE**. CO2 Hydrogenation to Ethanol over Cu@Na-Beta. *Chem.*, 2020, vol. 6, 2673-2689, https://doi.org/10.1016/j.chempr.2020.07.001
- **D. XU** ; **H. YANG** ; **X. HONG** ; **G. LIU** ; **S.C. EDMAN TSANG**. Tandem Catalysis of Direct CO2 Hydrogenation to Higher Alcohols. *ACS Catal.*, 2021, vol. 11, 8978-8984
- **D. XU** ; **M. DING** ; **X. HONG** ; **G. LIU**. Mechanistic aspects of the role of K promotion on Cu-Fe-based catalysts for higher alcohol synthesis from CO2 hydrogenation. *ACS Catal.*, 2020, vol. 10, 14516-14526, https://doi.org/10.1021/acscatal.0c03575
- **G. ZHANG** ; **G. FAN** ; **L. ZHENG** ; **F. LI**. Ga-Promoted CuCo-Based Catalysts for Efficient CO2 Hydrogenation to Ethanol: The Key Synergistic Role of Cu-CoGaOxInterfacial Sites. *ACS Appl. Mater. interfaces.*, 2022, vol. 14, 35569-35580
- **H. GUO** ; **S. LI** ; **F. PENG** ; **H. ZHANG** ; **L. XIONG** ; **C. HUANG** ; **C. WANG** ; **X. CHEN**. Roles Investigation of Promoters in K/Cu-Zn Catalyst and Higher Alcohols Synthesis from CO2 Hydrogenation over a Novel Two-Stage Bed Catalyst Combination System. *Catal. Letters.*, 2015, vol. 145, 620-630, https://doi.org/10.1007/s10562-014-1446-7

- **S. SANTANTA** ; **M.T.M. KOPER** ; **H.M. ALISSON** ; **L.H. VIEIRA** ; **E.M. ASSAF** ; **M. ASSAF** ; **J.F. GOMES**. Ethanol formation from CO2 hydrogenation at atmospheric pressure using Cu catalysts: Water as a key component. *Appl. Catal. B, Environ.*, 2023, vol. 324, 122221, https://doi.org/10.1016/j.apcatb.2022.122221
- **M. TAKAGAWA** ; **A. OKAMOTO** ; **H. FUJIMURA** ; **Y. IZAWA** ; **H. ARAKAWA**. Ethanol synthesis from carbon dioxide and hydrogen. *Stud. Surf. Sci. Catal.*, 1998, vol. 114, 525-528, https://doi.org/10.1016/s0167-2991(98)80812-4

- **Z. SI** ; **L. WANG** ; **Y. HAN** ; **J. YU** ; **Q. GE** ; **C. ZENG** ; **J. SUN**. Synthesis of Alkene and Ethanol in CO2 Hydrogenation on a Highly Active Sputtering CuNaFe Catalyst, ACS Sustain. *Chem. Eng.*, 2022, vol. 10, 14972-14979, https://doi.org/10.1021/acssuschemeng.2c05450
- **Y. WANG** ; **X. ZHANG** ; **X. HONG** ; **G. LIU**. Sulfate-Promoted Higher Alcohol Synthesis from CO2 Hydrogenation, ACS Sustain. *Chem. Eng.*, 2022, vol. 10, 8980-8987, https://doi.org/10.1021/acssuschemeng.2c02743